# EUROPEAN PATENT SPECIFICATION

(11) **EP 1 899 487 B1**
(45) Date of publication and mention of the grant of the patent: **20.04.2011**
(21) Application number: 06785181.6
(22) Date of filing: 20.06.2006
(51) Int. Cl.: G01N 33/50, C12Q 1/48, G01N 33/574, A61K 39/00, A61K 48/00

(54) **GFATS AS MODIFIERS OF THE AXIN PATHWAY AND METHODS OF USE**
GFATS ALS MODIFIKATOREN DES AXIN-WEGS UND VERWENDUNGSVERFAHREN
GFATS UTILISÉS COMME MODIFICATEURS DE LA VOIE DE L'AXINE ET LEURS PROCÉDÉS D'UTILISATION

(30) Priority: 20.06.2005 US 692316 P
(43) Date of publication of application: 19.03.2008
(73) Proprietor: Exelixis, Inc., South San Francisco, CA 94083-0511 (US)
(72) Inventor: GENDREAU, Steven, Brian, San Francisco, CA 94115 (US); DORA, Emery, G.III, Chapel Hill, NC 27517 (US); LICKTEIG, Kim, Illinois 60035 (US)
(74) Representative: Main, Malcolm Charles
(86) International application number: PCT/US2006/023971
(87) International publication number: WO 2007/002081

(56) References cited:
- WO-A-03/043631
- WO-A2-2004/013308
- WO-A2-2004/013309
- WO-A2-2004/047754
- WO-A2-2004/061086
- WO-A2-2004/066948
- WO-A2-2004/072257
- US-A1- 2004 102 412
- US-A1- 2004 132 020
- GELLER ET AL.: 'Inhibition of Gene Expression in Escherichia coli by Antisense Phosphorodiamidate Morpholino Oligomers' ANTIMICROBIAL AGENTS AND CHEMOTHERAPY 2003, pages 3233 - 3239, XP002402831

## Description

### BACKGROUND OF THE INVENTION

Deregulation of beta-catenin signaling is a frequent and early event in the development of a variety of human tumors, including colon cancer, melanoma, ovarian cancer, and prostate cancer. Activation of beta-catenin signaling can occur in tumor cells by loss-of-function mutations in the tumor suppressor genes Axin or APC, as well as by gain-of-function mutations in the oncogene beta-catenin itself. Axin normally functions as a scaffolding protein that binds beta-catenin, APC, and the serine/threonine kinase GSK3-beta. Assembly of this degradation complex allows GSK3-beta to phosphorylate beta-catenin, which leads to beta-catenin ubiquitination and degradation by the proteasome. In the absence of Axin activity, beta-catenin protein becomes stabilized and accumulates in the nucleus where it acts as a transcriptional co-activator with TCF for the induction of target genes, including the cell cycle regulators cyclin D1 and c-Myc.

The C. elegans gene pry-1 is the structural and functional ortholog of vertebrate Axin (Korswagen HC et al. (2002) Genes Dev. 16:1291-302). PRY-1 is predicted to contain conserved RGS and DIX domains that, in Axin, bind APC and Dishevelled, respectively. Overexpression of the C. elegans pry-1 gene in zebrafish can fully rescue the mutant phenotype of masterblind, the zebrafish Axin1 mutation. pry-1 loss-of-function mutations produce several phenotypes that appear to result from increased beta-catenin signaling (Gleason JE et al. (2002) Genes Dev. 16:1281-90; Korswagen et al., supra).

Glutamine:fructose-6-phosphate amidotransferases (GFATs or GFPTs) are involved in the hexosamine bisynthesis pathway. GFAT1 or GFPT1 intiates the formation of glucosamine 6-phosphate, the first step as well as the rate-limiting enzyme of the hexosamine biosynthetic pathway (Traxinger, R., and Marshall, S. (1991) J. Biol. Chem. 266: 10148-10154). GFAT1 controls the flux of glucose into the hexosamine pathway, and therefore controls the formation of hexosamine products. GFAT1 is likely to be involved in regulating the availability of precursors for N- and O-linked glycosylation of proteins (Robinson, A. et al. (1993) Diabetes. 42: 1333-1346). It is an insulin-regulated enzyme, plays an important role in the induction of insulin resistance in cultured cells, and is involved in the upregulation in kidney associated with diabetic nephropathy (Daniels, M. et al. (1996) J Clin Invest; 97(5): 1235-41). In MDA468 human breast cells, EGF stimulates the accumulation of GFAT messenger RNA (mRNA) to a level 4-fold higher than that in unstimulated cells (Paterson AJ, and Kudlow JE. (1995) Endocrinology 136:2809-2816).

Glutamine-fructose-6-phosphate transaminase 2 (GFAT2 or GFPT2) forms glucosamine 6-phosphate by transferring the amide group from L-glutamine to fructose 6-phosphate in the synthesis of hexosamines. Add stuff related to the gene of interest here.

The ability to manipulate the genomes of model organisms such as C. elegans provides a powerful means to analyze biochemical processes that, due to significant evolutionary conservation, have direct relevance to more complex vertebrate organisms. Due to a high level of gene and pathway conservation, the strong similarity of cellular processes, and the functional conservation of genes between these model organisms and mammals, identification of the involvement of novel genes in particular pathways and their functions in such model organisms can directly contribute to the understanding of the correlative pathways and methods of modulating them in mammals (see, for example, Dulubova I, et al, J Neurochem 2001 Apr;77(1):229-38; Cai T, et al., Diabetologia 2001 Jan;44(1):81-8; Pasquinelli AE, et al., Nature. 2000 Nov 2;408(6808):37-8; Ivanov IP, et al., EMBO J 2000 Apr 17;19(8):1907-17; Vajo Z et al., Mamm Genome 1999 Oct;10(10):1000-4). For example, a genetic screen can be carried out in an invertebrate model organism having underexpression (e.g. knockout) or overexpression of a gene (referred to as a "genetic entry point") that yields a visible phenotype. Additional genes are mutated in a random or targeted manner. When a gene mutation changes the original phenotype caused by the mutation in the genetic entry point, the gene is identified as a "modifier" involved in the same or overlapping pathway as the genetic entry point. When the genetic entry point is an ortholog of a human gene implicated in a disease pathway, such as axin, modifier genes can be identified that may be attractive candidate targets for novel therapeutics.

All references cited herein, including patents, patent applications, publications, and sequence information in referenced Genbank identifier numbers, are incorporated herein in their entireties.

### SUMMARY OF THE INVENTION

We have discovered genes that modify the Axin pathway in *C. elegans,* and identified their human orthologs, hereinafter referred to as Glutamine:fructose-6-phosphate amidotransferases (GFAT). The invention provides methods for utilizing these Axin modifier genes and polypeptides to identify GFAT-modulating agents that are candidate therapeutic agents that can be used in the treatment of disorders associated with defective or impaired Axin function and/or GFAT function. Preferred GFAT-modulating agents specifically bind to GFAT polypeptides and restore Axin function. Other preferred GFAT-modulating agents are nucleic acid modulators such as antisense oligomers and RNAi that repress GFAT gene expression or product activity by, for example, binding to and inhibiting the respective nucleic acid (i.e. DNA or mRNA). The invention provides methods and medical uses as defined by the claims.

GFAT modulating agents may be evaluated by any convenient *in vitro* or *in vivo* assay for molecular interaction with a GFAT polypeptide or nucleic acid. In one embodiment, candidate GFAT modulating agents are tested with an assay system comprising a GFAT polypeptide or nucleic acid. Agents that produce a change in the activity of the assay system relative to controls are identified as candidate Axin modulating agents. The assay system may be cell-based or cell-free. GFAT-modulating agents include GFAT related proteins (e.g. dominant negative mutants, and biotherapeutics); GFAT-specific antibodies; GFAT -specific antisense oligomers and other nucleic acid modulators; and chemical agents that specifically bind to or interact with GFAT or compete with GFAT binding partner (e.g. by binding to a GFAT binding partner). In one specific embodiment, a small molecule modulator is identified using a trasferase assay. In specific embodiments, the screening assay system is selected from a binding assay, an apoptosis assay, a cell proliferation assay, an angiogenesis assay, and a hypoxic induction assay.

In another embodiment, candidate Axin pathway modulating agents are further tested using a second assay system that detects changes in the Axin pathway, such as angiogenic, apoptotic, or cell proliferation changes produced by the originally identified candidate agent or an agent derived from the original agent. The second assay system may use cultured cells or non-human animals. In specific embodiments, the secondary assay system uses non-human animals, including animals predetermined to have a disease or disorder implicating the Axin pathway, such as an angiogenic, apoptotic, or cell proliferation disorder (e.g. cancer).

The invention further provides in vitro methods for modulating the GFAT function and/or the Axin pathway in a mammalian cell by contacting the mammalian cell with an agent that specifically binds a GFAT polypeptide or nucleic acid as defined by claims 11-12.

### DETAILED DESCRIPTION OF THE INVENTION

Genetic screens were designed to identify modifiers of the axin pathway in C. elegans, where a reduction of function pry-1 (axin) mutant was used. Various specific genes were silenced by RNA inhibition (RNAi). Methods for using RNAi to silence genes in C. elegans are known in the art (Fire A, et al., 1998 Nature 391:806-811; Fire, A. Trends Genet. 15, 358-363 (1999); WO9932619). Genes causing altered phenotypes in the worms were identified as modifiers of the Axin pathway. A modifier of particular interest, F07A11.2, was identified followed by identification of its orthologs. Accordingly, vertebrate orthologs of the modifier, and preferably the human orthologs, GFAT genes (i.e., nucleic acids and polypeptides) are attractive drug targets for the treatment of pathologies associated with a defective Axin signaling pathway, such as cancer.

In vitro and in vivo methods of assessing GFAT function are disclosed herein. Modulation of the GFAT or their respective binding partners is useful for understanding the association of the Axin pathway and its members in normal and disease conditions and for developing diagnostics and therapeutic modalities for Axin related pathologies. GFAT-modulating agents that act by inhibiting or enhancing GFAT expression, directly or indirectly, for example, by affecting a GFAT function such as enzymatic (e.g., catalytic) or binding activity, can be identified using methods provided herein. GFAT modulating agents are useful in diagnosis, therapy and pharmaceutical development.

### Nucleic acids and polypeptides of the invention

Sequences related to GFAT nucleic acids and polypeptides that can be used in the invention are disclosed in Genbank (referenced by Genbank identifier (GI) number) as GI#s 183081 (SEQ ID NO:1), 4503980 (SEQ ID NO:2), 4826741 (SEQ ID NO:3), 10433934 (SEQ ID NO:4), and 12652544 (SEQ ID NO:5) for nucleic acid, and GI#s 544382 (SEQ ID NO:6), 4503981 (SEQ ID NO:7), and 4826742 (SEQ ID NO:8) for polypeptides.

GFATs are transferase proteins with amidotransferase and sugar isomerase (SIS) domains. The term "GFAT polypeptide" refers to a full-length GFAT protein or a functionally active fragment or derivative thereof. A "functionally active" GFAT fragment or derivative exhibits one or more functional activities associated with a full-length, wild-type GFAT protein, such as antigenic or immunogenic activity, enzymatic activity, ability to bind natural cellular substrates, etc. The functional activity of GFAT proteins, derivatives and fragments can be assayed by various methods known to one skilled in the art (Current Protocols in Protein Science (1998) Coligan et al., eds., John Wiley & Sons, Inc., Somerset, New Jersey) and as further discussed below. In one embodiment, a functionally active GFAT polypeptide is a GFAT derivative capable of rescuing defective endogenous GFAT activity, such as in cell based or animal assays; the rescuing derivative may be from the same or a different species. For purposes herein, functionally active fragments also include those fragments that comprise one or more structural domains of a GFAT, such as a binding domain. Protein domains can be identified using the PFAM program (Bateman A., et al., Nucleic Acids Res, 1999, 27:260-2). For example, the Glutamine amidotransferases class-II domains(PFAM 00310) of GFATs from GI#s 4503981 (SEQ ID NO:7) and 4826742 (SEQ ID NO:8) are located at approximately amino acid residues 2-263 and 2-155, respectively. Likewise, the SIS domain (PFAM01380) of GFATs from GI#s 4503981 (SEQ ID NO:7) and 4826742 (SEQ ID NO:8) are located at approximately amino acid residues 360 to 494, 531 to 667 for SEQ ID NO:7 and 361 to 495, 532 to 668 for SEQ ID NO:8, respectively. Methods for obtaining GFAT polypeptides are also further described below. In some embodiments, preferred fragments are functionally active, domain-containing fragments comprising at least 25 contiguous amino acids, preferably at least 50, more preferably 75, and most preferably at least 100 contiguous amino acids of a GFAT. In further preferred embodiments, the fragment comprises the entire functionally active domain.

The term "GFAT nucleic acid" refers to a DNA or RNA molecule that encodes a GFAT polypeptide. Preferably, the GFAT polypeptide or nucleic acid or fragment thereof is from a human, but can also be an ortholog, or derivative thereof with at least 70% sequence identity, preferably at least 80%, more preferably 85%, still more preferably 90%, and most preferably at least 95% sequence identity with human GFAT. Methods of identifying orthlogs are known in the art. Normally, orthologs in different species retain the same function, due to presence of one or more protein motifs and/or 3-dimensional structures. Orthologs are generally identified by sequence homology analysis, such as BLAST analysis, usually using protein bait sequences. Sequences are assigned as a potential ortholog if the best hit sequence from the forward BLAST result retrieves the original query sequence in the reverse BLAST (Huynen MA and Bork P, Proc Natl Acad Sci (1998) 95:5849-5856; Huynen MA et al., Genome Research (2000) 10:1204-1210). Programs for multiple sequence alignment, such as CLUSTAL (Thompson JD et al, 1994, Nucleic Acids Res 22:4673-4680) may be used to highlight conserved regions and/or residues of orthologous proteins and to generate phylogenetic trees. In a phylogenetic tree representing multiple homologous sequences from diverse species (e.g., retrieved through BLAST analysis), orthologous sequences from two species generally appear closest on the tree with respect to all other sequences from these two species. Structural threading or other analysis of protein folding (e.g., using software by ProCeryon, Biosciences, Salzburg, Austria) may also identify potential orthologs. In evolution, when a gene duplication event follows speciation, a single gene in one species, such as *C. elegans,* may correspond to multiple genes (paralogs) in another, such as human. As used herein, the term "orthologs" encompasses paralogs. As used herein, "percent (%) sequence identity" with respect to a subject sequence, or a specified portion of a subject sequence, is defined as the percentage of nucleotides or amino acids in the candidate derivative sequence identical with the nucleotides or amino acids in the subject sequence (or specified portion thereof), after aligning the sequences and introducing gaps, if necessary to achieve the maximum percent sequence identity, as generated by the program WU-BLAST-2.0a19 (Altschul et al., J. Mol. Biol. (1997) 215:403-410) with all the search parameters set to default values. The HSP S and HSP S2 parameters are dynamic values and are established by the program itself depending upon the composition of the particular sequence and composition of the particular database against which the sequence of interest is being searched. A % identity value is determined by the number of matching identical nucleotides or amino acids divided by the sequence length for which the percent identity is being reported. "Percent (%) amino acid sequence similarity" is determined by doing the same calculation as for determining % amino acid sequence identity, but including conservative amino acid substitutions in addition to identical amino acids in the computation.

A conservative amino acid substitution is one in which an amino acid is substituted for another amino acid having similar properties such that the folding or activity of the protein is not significantly affected. Aromatic amino acids that can be substituted for each other are phenylalanine, tryptophan, and tyrosine; interchangeable hydrophobic amino acids are leucine, isoleucine, methionine, and valine; interchangeable polar amino acids are glutamine and asparagine; interchangeable basic amino acids are arginine, lysine and histidine; interchangeable acidic amino acids are aspartic acid and glutamic acid; and interchangeable small amino acids are alanine, serine, threonine, cysteine and glycine.

Alternatively, an alignment for nucleic acid sequences is provided by the local homology algorithm of Smith and Waterman (Smith and Waterman, 1981, Advances in Applied Mathematics 2:482-489; database: European Bioinformatics Institute; Smith and Waterman, 1981, J. of Molec.Biol., 147:195-197; Nicholas et al., 1998, "A Tutorial on Searching Sequence Databases and Sequence Scoring Methods" (www.psc.edu) and references cited therein.; W.R. Pearson, 1991, Genomics 11:635-650). This algorithm can be applied to amino acid sequences by using the scoring matrix developed by Dayhoff (Dayhoff: Atlas of Protein Sequences and Structure, M. O. Dayhoff ed., 5 suppl. 3:353-358, National Biomedical Research Foundation, Washington, D.C., USA), and normalized by Gribskov (Gribskov 1986 Nucl. Acids Res. 14(6):6745-6763). The Smith-Waterman algorithm may be employed where default parameters are used for scoring (for example, gap open penalty of 12, gap extension penalty of two). From the data generated, the "Match" value reflects "sequence identity."

Derivative nucleic acid molecules of the subject nucleic acid molecules include sequences that hybridize to the nucleic acid sequence of a GFAT. The stringency of hybridization can be controlled by temperature, ionic strength, pH, and the presence of denaturing agents such as formamide during hybridization and washing. Conditions routinely used are set out in readily available procedure texts (*e.g*., Current Protocol in Molecular Biology, Vol. 1, Chap. 2.10, John Wiley & Sons, Publishers (1994); Sambrook et al., Molecular Cloning, Cold Spring Harbor (1989)). In some embodiments, a nucleic acid molecule of the invention is capable of hybridizing to a nucleic acid molecule containing the nucleotide sequence of a GFAT under high stringency hybridization conditions that are: prehybridization of filters containing nucleic acid for 8 hours to overnight at 65° C in a solution comprising 6X single strength citrate (SSC) (1X SSC is 0.15 M NaCl, 0.015 M Na citrate; pH 7.0), 5X Denhardt's solution, 0.05% sodium pyrophosphate and 100 µg/ml herring sperm DNA; hybridization for 18-20 hours at 65° C in a solution containing 6X SSC, 1X Denhardt's solution, 100 µg/ml yeast tRNA and 0.05% sodium pyrophosphate; and washing of filters at 65° C for 1h in a solution containing 0.1X SSC and 0.1% SDS (sodium dodecyl sulfate).

In other embodiments, moderately stringent hybridization conditions are used that are: pretreatment of filters containing nucleic acid for 6 h at 40° C in a solution containing 35% formamide, 5X SSC, 50 mM Tris-HCl (pH7.5), 5mM EDTA, 0.1% PVP, 0.1% Ficoll, 1% BSA, and 500 µg/ml denatured salmon sperm DNA; hybridization for 18-20h at 40° C in a solution containing 35% formamide, 5X SSC, 50 mM Tris-HCl (pH7.5), 5mM EDTA, 0.02% PVP, 0.02% Ficoll, 0.2% BSA, 100 µg/ml salmon sperm DNA, and 10% (wt/vol) dextran sulfate; followed by washing twice for 1 hour at 55° C in a solution containing 2X SSC and 0.1 % SDS.

Alternatively, low stringency conditions can be used that are: incubation for 8 hours to overnight at 37° C in a solution comprising 20% formamide, 5 x SSC, 50 mM sodium phosphate (pH 7.6), 5X Denhardt's solution, 10% dextran sulfate, and 20 µg/ml denatured sheared salmon sperm DNA; hybridization in the same buffer for 18 to 20 hours; and washing of filters in 1 x SSC at about 37° C for 1 hour.

### Isolation, Production, Expression, and Mis-expression of GFAT Nucleic Acids and Polypeptides

GFAT nucleic acids and polypeptides are useful for identifying and testing agents that modulate GFAT function and for other applications related to the involvement of GFAT in the Axin pathway. GFAT nucleic acids and derivatives and orthologs thereof may be obtained using any available method. For instance, techniques for isolating cDNA or genomic DNA sequences of interest by screening DNA libraries or by using polymerase chain reaction (PCR) are well known in the art. In general, the particular use for the protein will dictate the particulars of expression, production, and purification methods. For instance, production of proteins for use in screening for modulating agents may require methods that preserve specific biological activities of these proteins, whereas production of proteins for antibody generation may require structural integrity of particular epitopes. Expression of proteins to be purified for screening or antibody production may require the addition of specific tags (*e.g*., generation of fusion proteins). Overexpression of a GFAT protein for assays used to assess GFAT function, such as involvement in cell cycle regulation or hypoxic response, may require expression in eukaryotic cell lines capable of these cellular activities. Techniques for the expression, production, and purification of proteins are well known in the art; any suitable means therefore may be used (e.g., Higgins SJ and Hames BD (eds.) Protein Expression: A Practical Approach, Oxford University Press Inc., New York 1999; Stanbury PF et al., Principles of Fermentation Technology, 2nd edition, Elsevier Science, New York, 1995; Doonan S (ed.) Protein Purification Protocols, Humana Press, New Jersey, 1996; Coligan JE et al, Current Protocols in Protein Science (eds.), 1999, John Wiley & Sons, New York). In particular embodiments, recombinant GFAT is expressed in a cell line known to have defective Axin function. The recombinant cells are used in cell-based screening assay systems of the invention, as described further below.

The nucleotide sequence encoding a GFAT polypeptide can be inserted into any appropriate expression vector. The necessary transcriptional and translational signals, including promoter/enhancer element, can derive from the native GFAT gene and/or its flanking regions or can be heterologous. A variety of host-vector expression systems may be utilized, such as mammalian cell systems infected with virus (*e.g*. vaccinia virus, adenovirus, *etc*.); insect cell systems infected with virus (*e.g*. baculovirus); microorganisms such as yeast containing yeast vectors, or bacteria transformed with bacteriophage, plasmid, or cosmid DNA. An isolated host cell strain that modulates the expression of, modifies, and/or specifically processes the gene product may be used.

To detect expression of the GFAT gene product, the expression vector can comprise a promoter operably linked to a GFAT gene nucleic acid, one or more origins of replication, and, one or more selectable markers (*e.g*. thymidine kinase activity, resistance to antibiotics, *etc*.). Alternatively, recombinant expression vectors can be identified by assaying for the expression of the GFAT gene product based on the physical or functional properties of the GFAT protein in *in vitro* assay systems (*e.g*. immunoassays).

The GFAT protein, fragment, or derivative may be optionally expressed as a fusion, or chimeric protein product (i.e. it is joined via a peptide bond to a heterologous protein sequence of a different protein), for example to facilitate purification or detection. A chimeric product can be made by ligating the appropriate nucleic acid sequences encoding the desired amino acid sequences to each other using standard methods and expressing the chimeric product. A chimeric product may also be made by protein synthetic techniques, *e.g*. by use of a peptide synthesizer (Hunkapiller et al., Nature (1984) 310:105-111).

Once a recombinant cell that expresses the GFAT gene sequence is identified, the gene product can be isolated and purified using standard methods (*e.g*. ion exchange, affinity, and gel exclusion chromatography; centrifugation; differential solubility; electrophoresis). Alternatively, native GFAT proteins can be purified from natural sources, by standard methods (*e.g*. immunoaffinity purification). Once a protein is obtained, it may be quantified and its activity measured by appropriate methods, such as immunoassay, bioassay, or other measurements of physical properties, such as crystallography.

The methods of this invention may also use cells that have been engineered for altered expression (mis-expression) of GFAT or other genes associated with the Axin pathway. As used herein, mis-expression encompasses ectopic expression, overexpression, under-expression, and non-expression (*e.g*. by gene knock-out or blocking expression that would otherwise normally occur).

### Genetically modified animals

Animal models that have been genetically modified to alter GFAT expression may be used in *in vivo* assays to test for activity of a candidate Axin modulating agent, or to further assess the role of GFAT in a Axin pathway process such as apoptosis or cell proliferation (not part of the invention). Preferably, the altered GFAT expression results in a detectable phenotype, such as decreased or increased levels of cell proliferation, angiogenesis, or apoptosis compared to control animals having normal GFAT expression. The genetically modified animal may additionally have altered Axin expression (e.g. Axin knockout). Preferred genetically modified animals are mammals such as primates, rodents (preferably mice or rats), among others. Preferred non-mammalian species include zebrafish, *C*. *elegans,* and *Drosophila.* Preferred genetically modified animals are transgenic animals having a heterologous nucleic acid sequence present as an extrachromosomal element in a portion of its cells, i.e. mosaic animals (see, for example, techniques described by Jakobovits, 1994, Curr. Biol. 4:761-763.) or stably integrated into its germ line DNA (i.e., in the genomic sequence of most or all of its cells). Heterologous nucleic acid is introduced into the germ line of such transgenic animals by genetic manipulation of, for example, embryos or embryonic stem cells of the host animal.

Methods of making transgenic animals are well-known in the art (for transgenic mice see Brinster et al., Proc. Nat. Acad. Sci. USA 82: 4438-4442 (1985), U.S. Pat. Nos. 4,736,866 and 4,870,009, both by Leder et al., U.S. Pat. No. 4,873,191 by Wagner et al., and Hogan, B., Manipulating the Mouse Embryo, Cold Spring Harbor Laboratory Press, Cold Spring Harbor, N.Y., (1986); for particle bombardment see U.S. Pat. No., 4,945,050, by Sandford et al.*;* for transgenic *Drosophila* see Rubin and Spradling, Science (1982) 218:348-53 and U.S. Pat. No. 4,670,388; for transgenic insects see Berghammer A.J. et al., A Universal Marker for Transgenic Insects (1999) Nature 402:370-371; for transgenic Zebrafish see Lin S., Transgenic Zebrafish, Methods Mol Biol. (2000);136:375-3830); for microinjection procedures for fish, amphibian eggs and birds see Houdebine and Chourrout, Experientia (1991) 47:897-905; for transgenic rats see Hammer et al., Cell (1990) 63:1099-1112; and for culturing of embryonic stem (ES) cells and the subsequent production of transgenic animals by the introduction of DNA into ES cells using methods such as electroporation, calcium phosphate/DNA precipitation and direct injection see, e.g., Teratocarcinomas and Embryonic Stem Cells, A Practical Approach, E. J. Robertson, ed., IRL Press (1987)). Clones of the nonhuman transgenic animals can be produced according to available methods (see Wilmut, I. et al. (1997) Nature 385:810-813; and PCT International Publication Nos. WO 97/07668 and WO 97/07669).

The transgenic animal may be a "knock-out" animal having a heterozygous or homozygous alteration in the sequence of an endogenous GFAT gene that results in a decrease of GFAT function, preferably such that GFAT expression is undetectable or insignificant. Knock-out animals are typically generated by homologous recombination with a vector comprising a transgene having at least a portion of the gene to be knocked out. Typically a deletion, addition or substitution has been introduced into the transgene to functionally disrupt it. The transgene can be a human gene (e.g., from a human genomic clone) but more preferably is an ortholog of the human gene derived from the transgenic host species. For example, a mouse GFAT gene is used to construct a homologous recombination vector suitable for altering an endogenous GFAT gene in the mouse genome. Detailed methodologies for homologous recombination in mice are available (see Capecchi, Science (1989) 244:1288-1292; Joyner et al., Nature (1989) 338:153-156). Procedures for the production of non-rodent transgenic mammals and other animals are also available (Houdebine and Chourrout, *supra;* Pursel et al., Science (1989) 244:1281-1288; Simms et al., Bio/Technology (1988) 6:179-183). In a preferred embodiment, knock-out animals, such as mice harboring a knockout of a specific gene, may be used to produce antibodies against the human counterpart of the gene that has been knocked out (Claesson MH et al., (1994) Scan J Immunol 40:257-264; Declerck PJ et al., (1995) J Biol Chem. 270:8397-400).

The transgenic animal may be a "knock-in" animal having an alteration in its genome that results in altered expression (e.g., increased (including ectopic) or decreased expression) of the GFAT gene, e.g., by introduction of additional copies of GFAT, or by operatively inserting a regulatory sequence that provides for altered expression of an endogenous copy of the GFAT gene. Such regulatory sequences include inducible, tissue-specific, and constitutive promoters and enhancer elements. The knock-in can be homozygous or heterozygous.

Transgenic nonhuman animals can also be produced that contain selected systems allowing for regulated expression of the transgene. One example of such a system that may be produced is the cre/loxP recombinase system of bacteriophage P1 (Lakso et al., PNAS (1992) 89:6232-6236; U.S. Pat. No. 4,959,317). If a cre/loxP recombinase system is used to regulate expression of the transgene, animals containing transgenes encoding both the Cre recombinase and a selected protein are required. Such animals can be provided through the construction of "double" transgenic animals, e.g., by mating two transgenic animals, one containing a transgene encoding a selected protein and the other containing a transgene encoding a recombinase. Another example of a recombinase system is the FLP recombinase system of Saccharomyces cerevisiae (O'Gorman et al. (1991) Science 251:1351-1355; U.S. Pat. No. 5,654,182). In a preferred embodiment, both Cre-LoxP and Flp-Frt are used in the same system to regulate expression of the transgene, and for sequential deletion of vector sequences in the same cell (Sun X et al (2000) Nat Genet 25:83-6).

The genetically modified animals can be used in genetic studies to further elucidate the Axin pathway, as animal models of disease and disorders implicating defective Axin function, and for *in vivo* testing of candidate therapeutic agents, such as those identified in screens described below. The candidate therapeutic agents are administered to a genetically modified animal having altered GFAT function and phenotypic changes are compared with appropriate control animals such as genetically modified animals that receive placebo treatment, and/or animals with unaltered GFAT expression that receive candidate therapeutic agent.

In addition to the above-described genetically modified animals having altered GFAT function, animal models having defective Axin function (and otherwise normal GFAT function), can be used in the methods disclosed by the present invention. For example, a Axin knockout mouse can be used to assess, *in vivo,* the activity of a candidate Axin modulating agent identified in one of the *in vitro* assays described below. Preferably, the candidate Axin modulating agent when administered to a model system with cells defective in Axin function, produces a detectable phenotypic change in the model system indicating that the Axin function is restored, i.e., the cells exhibit normal cell cycle progression.

### Modulating Agents

The invention provides methods to identify agents that interact with and/or modulate the function of the Axin pathway. Modulating agents identified by the methods are also part of the invention. Such agents are useful in a variety of diagnostic and therapeutic applications associated with the Axin pathway, as well as in further analysis of the GFAT protein and its contribution to the Axin pathway. Accordingly, the invention also provides methods for modulating the Axin pathway comprising the step of specifically modulating GFAT activity by administering a GFAT-interacting or -modulating agent.

As used herein, a "GFAT-modulating agent" is any agent that modulates GFAT function, for example, an agent that interacts with GFAT to inhibit or enhance GFAT activity or otherwise affect normal GFAT function. GFAT function can be affected at any level, including transcription, protein expression, protein localization, and cellular or extra-cellular activity. In a preferred embodiment, the GFAT - modulating agent specifically modulates the function of the GFAT. The phrases "specific modulating agent", "specifically modulates", etc., are used herein to refer to modulating agents that directly bind to the GFAT polypeptide or nucleic acid, and preferably inhibit, enhance, or otherwise alter, the function of the GFAT. These phrases also encompass modulating agents that alter the interaction of the GFAT with a binding partner, substrate, or cofactor (e.g. by binding to a binding partner of a GFAT, or to a protein/binding partner complex, and altering GFAT function). In a further preferred embodiment, the GFAT- modulating agent is a modulator of the Axin pathway (e.g. it restores and/or upregulates Axin function) and thus is also a Axin-modulating agent.

Preferred GFAT-modulating agents include small molecule compounds; GFAT-interacting proteins, including antibodies and other biotherapeutics; and nucleic acid modulators such as antisense and RNA inhibitors. The modulating agents may be formulated in pharmaceutical compositions, for example, as compositions that may comprise other active ingredients, as in combination therapy, and/or suitable carriers or excipients. Techniques for formulation and administration of the compounds may be found in "Remington's Pharmaceutical Sciences" Mack Publishing Co., Easton, PA, 19th edition.

### Small molecule modulators

Small molecules are often preferred to modulate function of proteins with enzymatic function, and/or containing protein interaction domains. Chemical agents, referred to in the art as "small molecule" compounds are typically organic, non-peptide molecules, having a molecular weight up to 10,000, preferably up to 5,000, more preferably up to 1,000, and most preferably up to 500 daltons. This class of modulators includes chemically synthesized molecules, for instance, compounds from combinatorial chemical libraries. Synthetic compounds may be rationally designed or identified based on known or inferred properties of the GFAT protein or may be identified by screening compound libraries. Alternative appropriate modulators of this class are natural products, particularly secondary metabolites from organisms such as plants or fungi, which can also be identified by screening compound libraries for GFAT-modulating activity. Methods for generating and obtaining compounds are well known in the art (Schreiber SL, Science (2000) 151: 1964-1969; Radmann J and Gunther J, Science (2000) 151:1947-1948).

Small molecule modulators identified from screening assays, as described below, can be used as lead compounds from which candidate clinical compounds may be designed, optimized, and synthesized. Such clinical compounds may have utility in treating pathologies associated with the Axin pathway. The activity of candidate small molecule modulating agents may be improved several-fold through iterative secondary functional validation, as further described below, structure determination, and candidate modulator modification and testing. Additionally, candidate clinical compounds are generated with specific regard to clinical and pharmacological properties. For example, the reagents may be derivatized and re-screened using *in vitro* and *in vivo* assays to optimize activity and minimize toxicity for pharmaceutical development.

### Protein Modulators

Specific GFAT-interacting proteins are useful in a variety of diagnostic and therapeutic applications related to the Axin pathway and related disorders, as well as in validation assays for other GFAT-modulating agents. In a preferred embodiment, GFAT-interacting proteins affect normal GFAT function, including transcription, protein expression, protein localization, and cellular or extra-cellular activity. In another embodiment, GFAT-interacting proteins are useful in detecting and providing information about the function of GFAT proteins, as is relevant to Axin related disorders, such as cancer (e.g., for diagnostic means).

A GFAT-interacting protein may be endogenous, i.e. one that naturally interacts genetically or biochemically with a GFAT, such as a member of the GFAT pathway that modulates GFAT expression, localization, and/or activity. GFAT-modulators include dominant negative forms of GFAT-interacting proteins and of GFAT proteins themselves. Yeast two-hybrid and variant screens offer preferred methods for identifying endogenous GFAT-interacting proteins (Finley, R. L. et al. (1996) in DNA Cloning-Expression Systems: A Practical Approach, eds. Glover D. & Hames B. D (Oxford University Press, Oxford, England), pp. 169-203; Fashema SF et al., Gene (2000) 250:1-14; Drees BL Curr Opin Chem Biol (1999) 3:64-70; Vidal M and Legrain P Nucleic Acids Res (1999) 27:919-29; and U.S. Pat. No. 5,928,868). Mass spectrometry is an alternative preferred method for the elucidation of protein complexes (reviewed in, e.g., Pandley A and Mann M, Nature (2000) 405:837-846; Yates JR 3rd, Trends Genet (2000) 16:5-8).

An GFAT-interacting protein may be an exogenous protein, such as a GFAT-specific antibody or a T-cell antigen receptor (see, e.g., Harlow and Lane (1988) Antibodies, A Laboratory Manual, Cold Spring Harbor Laboratory; Harlow and Lane (1999) Using antibodies: a laboratory manual. Cold Spring Harbor, NY: Cold Spring Harbor Laboratory Press). GFAT antibodies are further discussed below.

In preferred embodiments, a GFAT-interacting protein specifically binds a GFAT protein. In alternative preferred embodiments, a GFAT-modulating agent binds a GFAT substrate, binding partner, or cofactor.

### Antibodies

In another embodiment, the protein modulator is a GFAT specific antibody agonist or antagonist. The antibodies have therapeutic and diagnostic utilities, and can be used in screening assays to identify GFAT modulators. The antibodies can also be used in dissecting the portions of the GFAT pathway responsible for various cellular responses and in the general processing and maturation of the GFAT.

Antibodies that specifically bind GFAT polypeptides can be generated using known methods. Preferably the antibody is specific to a mammalian ortholog of GFAT polypeptide, and more preferably, to human GFAT. Antibodies may be polyclonal, monoclonal (mAbs), humanized or chimeric antibodies, single chain antibodies, Fab fragments, F(ab').sub.2 fragments, fragments produced by a FAb expression library, anti-idiotypic (anti-Id) antibodies, and epitope-binding fragments of any of the above. Epitopes of GFAT which are particularly antigenic can be selected, for example, by routine screening of GFAT polypeptides for antigenicity or by applying a theoretical method for selecting antigenic regions of a protein (Hopp and Wood (1981), Proc. Natl. Acad. Sci. U.S.A. 78:3824-28; Hopp and Wood, (1983) Mol. Immunol. 20:483-89; Sutcliffe et al., (1983) Science 219:660-66) to the amino acid sequence of a GFAT. Monoclonal antibodies with affinities of 10⁸ M⁻¹ preferably 10⁹ M⁻¹ to 10¹⁰ M⁻¹, or stronger can be made by standard procedures as described (Harlow and Lane, *supra;* Goding (1986) Monoclonal. Antibodies: Principles and Practice (2d ed) Academic Press, New York; and U.S. Pat. Nos. 4,381,292; 4,451,570; and 4,618,577). Antibodies may be generated against crude cell extracts of GFAT or substantially purified fragments thereof. If GFAT fragments are used, they preferably comprise at least 10, and more preferably, at least 20 contiguous amino acids of a GFAT protein. In a particular embodiment, GFAT-specific antigens and/or immunogens are coupled to carrier proteins that stimulate the immune response. For example, the subject polypeptides are covalently coupled to the keyhole limpet hemocyanin (KLH) carrier, and the conjugate is emulsified in Freund's complete adjuvant, which enhances the immune response. An appropriate immune system such as a laboratory rabbit or mouse is immunized according to conventional protocols.

The presence of GFAT-specific antibodies is assayed by an appropriate assay such as a solid phase enzyme-linked immunosorbant assay (ELISA) using immobilized corresponding GFAT polypeptides. Other assays, such as radioimmunoassays or fluorescent assays might also be used.

Chimeric antibodies specific to GFAT polypeptides can be made that contain different portions from different animal species. For instance, a human immunoglobulin constant region may be linked to a variable region of a murine mAb, such that the antibody derives its biological activity from the human antibody, and its binding specificity from the murine fragment. Chimeric antibodies are produced by splicing together genes that encode the appropriate regions from each species (Morrison et al., Proc. Natl. Acad. Sci. (1984) 81:6851-6855; Neuberger et al., Nature (1984) 312:604-608; Takeda et al., Nature (1985) 31:452-454). Humanized antibodies, which are a form of chimeric antibodies, can be generated by grafting complementary-determining regions (CDRs) (Carlos, T. M., J. M. Harlan. 1994. Blood 84:2068-2101) of mouse antibodies into a background of human framework regions and constant regions by recombinant DNA technology (Riechmann LM, et al., 1988 Nature 323: 323-327). Humanized antibodies contain ~10% murine sequences and ~90% human sequences, and thus further reduce or eliminate immunogenicity, while retaining the antibody specificities (Co MS, and Queen C. 1991 Nature 351: 501-501; Morrison SL. 1992 Ann. Rev. Immun. 10:239-265). Humanized antibodies and methods of their production are well-known in the art (U.S. Pat. Nos. 5,530,101, 5,585,089, 5,693,762, and 6,180,370).

GFAT-specific single chain antibodies which are recombinant, single chain polypeptides formed by linking the heavy and light chain fragments of the Fv regions via an amino acid bridge, can be produced by methods known in the art (U.S. Pat. No. 4,946,778; Bird, Science (1988) 242:423-426; Huston et al., Proc. Natl. Acad. Sci. USA (1988) 85:5879-5883; and Ward et al., Nature (1989) 334:544-546).

Other suitable techniques for antibody production involve in vitro exposure of lymphocytes to the antigenic polypeptides or alternatively to selection of libraries of antibodies in phage or similar vectors (Huse et al., Science (1989) 246:1275-1281). As used herein, T-cell antigen receptors are included within the scope of antibody modulators (Harlow and Lane, 1988, *supra).*

The polypeptides and antibodies of the present invention may be used with or without modification. Frequently, antibodies will be labeled by joining, either covalently or non-covalently, a substance that provides for a detectable signal, or that is toxic to cells that express the targeted protein (Menard S, et al., Int J. Biol Markers (1989) 4:131-134). A wide variety of labels and conjugation techniques are known and are reported extensively in both the scientific and patent literature. Suitable labels include radionuclides, enzymes, substrates, cofactors, inhibitors, fluorescent moieties, fluorescent emitting lanthanide metals, chemiluminescent moieties, bioluminescent moieties, magnetic particles, and the like (U.S. Pat. Nos. 3,817,837; 3,850,752; 3,939,350; 3,996,345; 4,277,437; 4,275,149; and 4,366,241). Also, recombinant immunoglobulins may be produced (U.S. Pat. No. 4,816,567). Antibodies to cytoplasmic polypeptides may be delivered and reach their targets by conjugation with membrane-penetrating toxin proteins (U.S. Pat. No. 6,086,900).

When used therapeutically in a patient, the antibodies of the subject invention are typically administered parenterally, when possible at the target site, or intravenously. The therapeutically effective dose and dosage regimen is determined by clinical studies. Typically, the amount of antibody administered is in the range of about 0.1 mg/kg -to about 10 mg/kg of patient weight. For parenteral administration, the antibodies are formulated in a unit dosage injectable form (e.g., solution, suspension, emulsion) in association with a pharmaceutically acceptable vehicle. Such vehicles are inherently nontoxic and non-therapeutic. Examples are water, saline, Ringer's solution, dextrose solution, and 5% human serum albumin. Nonaqueous vehicles such as fixed oils, ethyl oleate, or liposome carriers may also be used. The vehicle may contain minor amounts of additives, such as buffers and preservatives, which enhance isotonicity and chemical stability or otherwise enhance therapeutic potential. The antibodies' concentrations in such vehicles are typically in the range of about 1 mg/ml to about10 mg/ml. Immunotherapeutic methods are further described in the literature (US Pat. No. 5,859,206; WO0073469).

### Nucleic Acid Modulators

Other preferred GFAT-modulating agents comprise nucleic acid molecules, such as antisense oligomers or double stranded RNA (dsRNA), which generally inhibit GFAT activity. Preferred nucleic acid modulators interfere with the function of the GFAT nucleic acid such as DNA replication, transcription, translocation of the GFATRNA to the site ot protein translation, translation of protein from the GFAT RNA, splicing of the GFAT RNA to yield one or more mRNA species, or catalytic activity which may be engaged in or facilitated by the GFAT RNA.

In one embodiment, the antisense oligomer is an oligonucleotide that is sufficiently complementary to a GFAT mRNA to bind to and prevent translation, preferably by binding to the 5' untranslated region. GFAT-specific antisense oligonucleotides, preferably range from at least 6 to about 200 nucleotides. In some embodiments the oligonucleotide is preferably at least 10, 15, or 20 nucleotides in length. In other embodiments, the oligonucleotide is preferably less than 50, 40, or 30 nucleotides in length. The oligonucleotide can be DNA or RNA or a chimeric mixture or derivatives or modified versions thereof, single-stranded or double-stranded. The oligonucleotide can be modified at the base moiety, sugar moiety, or phosphate backbone. The oligonucleotide may include other appending groups such as peptides, agents that facilitate transport across the cell membrane, hybridization-triggered cleavage agents, and intercalating agents.

In another embodiment, the antisense oligomer is a phosphothioate morpholino oligomer (PMO). PMOs are assembled from four different morpholino subunits, each of which contain one of four genetic bases (A, C, G, or T) linked to a six-membered morpholine ring. Polymers of these subunits are joined by non-ionic phosphodiamidate intersubunit linkages. Details of how to make and use PMOs and other antisense oligomers are well known in the art (e.g. see WO99/18193; Probst JC, Antisense Oligodeoxynucleotide and Ribozyme Design, Methods. (2000) 22(3):271-281; Summerton J, and Weller D. 1997 Antisense Nucleic Acid Drug Dev. :7:187-95; US Pat. No. 5,235,033; and US Pat No. 5,378,841).

Alternative preferred GFAT nucleic acid modulators are double-stranded RNA species mediating RNA interference (RNAi). RNAi is the process of sequence-specific, post-transcriptional gene silencing in animals and plants, initiated by double-stranded RNA (dsRNA) that is homologous in sequence to the silenced gene. Methods relating to the use of RNAi to silence genes in *C. elegans, Drosophila,* plants, and humans are known in the art (Fire A, et al., 1998 Nature 391:806-811; Fire, A. Trends Genet. 15, 358-363 (1999); Sharp, P. A. RNA interference 2001. Genes Dev. 15,485-490 (2001); Hammond, S. M., et al., Nature Rev. Genet. 2, 110-1119 (2001); Tuschl, T. Chem. Biochem. 2, 239-245 (2001); Hamilton, A. et al., Science 286, 950-952 (1999); Hammond, S. M., et al., Nature 404, 293-296 (2000); Zamore, P. D., et at., Cell 101, 25-33 (2000); Bernstein, E., et al., Nature 409, 363-366 (2001); Elbashir, S. M., et al., Genes Dev. 15, 188-200 (2001); WO0129058; WO9932619; Elbashir SM, et al., 2001 Nature 411:494-498; Novina CD and Sharp P. 2004 Nature 430:161-164; Soutschek J et al 2004 Nature 432:173-178; Hsieh AC et al. (2004) NAR 32(3):893-901).

Nucleic acid modulators are commonly used as research reagents, diagnostics, and therapeutics. For example, antisense oligonucleotides, which are able to inhibit gene expression with exquisite specificity, are often used to elucidate the function of particular genes (see, for example, U.S. Pat. No. 6,165,790). Nucleic acid modulators are also used, for example, to distinguish between functions of various members of a biological pathway. For example, antisense oligomers have been employed as therapeutic moieties in the treatment of disease states in animals and man and have been demonstrated in numerous clinical trials to be safe and effective (Milligan JF, et al, Current Concepts in Antisense Drug Design, J Med Chem. (1993) 36:1923-1937; Tonkinson JL et al., Antisense Oligodeoxynucleotides as Clinical Therapeutic Agents, Cancer Invest. (1996) 14:54-65). Accordingly, in one aspect of the invention, a GFAT-specific nucleic acid modulator is used in an assay to further elucidate the role of the GFAT in the Axin pathway, and/or its relationship to other members of the pathway. In another aspect of the invention, a GFAT-specific antisense oligomer is used as a therapeutic agent for treatment of Axin-related disease states.

### Assay Systems

The invention provides assay systems and screening methods for identifying specific modulators of GFAT activity. As used herein, an "assay system" encompasses all the components required for performing and analyzing results of an assay that detects and/or measures a particular event. In general, primary assays are used to identify or confirm a modulator's specific biochemical or molecular effect with respect to the GFAT nucleic acid or protein. In general, secondary assays further assess the activity of a GFAT modulating agent identified by a primary assay and may confirm that the modulating agent affects GFAT in a manner relevant to the Axin pathway. In some cases, GFAT modulators will be directly tested in a secondary assay.

In a preferred embodiment, the screening method comprises contacting a suitable assay system comprising a GFAT polypeptide or nucleic acid with a candidate agent under conditions whereby, but for the presence of the agent, the system provides a reference activity (e.g. transferase activity), which is based on the particular molecular event the screening method detects. A statistically significant difference between the agent-biased activity and the reference activity indicates that the candidate agent modulates GFAT activity, and hence the Axin pathway. The GFAT polypeptide or nucleic acid used in the assay may comprise any of the nucleic acids or polypeptides described above.

### Primary Assays

The type of modulator tested generally determines the type of primary assay.

### Primary assays for small molecule modulators

For small molecule modulators, screening assays are used to identify candidate modulators. Screening assays may be cell-based or may use a cell-free system that recreates or retains the relevant biochemical reaction of the target protein (reviewed in Sittampalam GS et al., Curr Opin Chem Biol (1997) 1:384-91 and accompanying references). As used herein the term "cell-based" refers to assays using live cells, dead cells, or a particular cellular fraction, such as a membrane, endoplasmic reticulum, or mitochondrial fraction. The term "cell free" encompasses assays using substantially purified protein (either endogenous or recombinantly produced), partially purified or crude cellular extracts. Screening assays may detect a variety of molecular events, including protein-DNA interactions, protein-protein interactions (*e.g.*, receptor-ligand binding), transcriptional activity (*e.g*., using a reporter gene), enzymatic activity (*e.g*., via a property of the substrate), activity of second messengers, immunogenicty and changes in cellular morphology or other cellular characteristics. Appropriate screening assays may use a wide range of detection methods including fluorescent, radioactive, colorimetric, spectrophotometric, and amperometric methods, to provide a read-out for the particular molecular event detected.

Cell-based screening assays usually require systems for recombinant expression of GFAT and any auxiliary proteins demanded by the particular assay. Appropriate methods for generating recombinant proteins produce sufficient quantities of proteins that retain their relevant biological activities and are of sufficient purity to optimize activity and assure assay reproducibility. Yeast two-hybrid and variant screens, and mass spectrometry provide preferred methods for determining protein-protein interactions and elucidation of protein complexes. In certain applications, when GFAT-interacting proteins are used in screens to identify small molecule modulators, the binding specificity of the interacting protein to the GFAT protein may be assayed by various known methods such as substrate processing (e.g. ability of the candidate GFAT-specific binding agents to function as negative effectors in GFAT-expressing cells), binding equilibrium constants (usually at least about 10⁷ M⁻¹, preferably at least about 10⁸ M⁻¹, more preferably at least about 10⁹ M⁻¹), and immunogenicity (e.g. ability to elicit GFAT specific antibody in a heterologous host such as a mouse, rat, goat or rabbit). For enzymes and receptors, binding may be assayed by, respectively, substrate and ligand processing.

The screening assay may measure a candidate agent's ability to specifically bind to or modulate activity of a GFAT polypeptide, a fusion protein thereof, or to cells or membranes bearing the polypeptide or fusion protein. The GFAT polypeptide can be full length or a fragment thereof that retains functional GFAT activity. The GFAT polypeptide may be fused to another polypeptide, such as a peptide tag for detection or anchoring, or to another tag. The GFAT polypeptide is preferably human GFAT, or is an ortholog or derivative thereof as described above. In a preferred embodiment, the screening assay detects candidate agent-based modulation of GFAT interaction with a binding target, such as an endogenous or exogenous protein or other substrate that has GFAT -specific binding activity, and can be used to assess normal GFAT gene function.

Suitable assay formats that may be adapted to screen for GFAT modulators are known in the art. Preferred screening assays are high throughput or ultra high throughput and thus provide automated, cost-effective means of screening compound libraries for lead compounds (Fernandes PB, Curr Opin Chem Biol (1998) 2:597-603; Sundberg SA, Curr Opin Biotechnol 2000, 11:47-53). In one preferred embodiment, screening assays uses fluorescence technologies, including fluorescence polarization, time-resolved fluorescence, and fluorescence resonance energy transfer. These systems offer means to monitor protein-protein or DNA-protein interactions in which the intensity of the signal emitted from dye-labeled molecules depends upon their interactions with partner molecules (*e.g*., Selvin PR, Nat Struct Biol (2000) 7:730-4; Fernandes PB, *supra;* Hertzberg RP and Pope AJ, Curr Opin Chem Biol (2000) 4:445-451).

A variety of suitable assay systems may be used to identify candidate GFAT and Axin pathway modulators (e.g. U.S. Pat. Nos. 5,550,019 and 6,133,437 (apoptosis assays); and U.S. Pat. Nos. 5,976,782, 6,225,118 and 6,444,434 (angiogenesis assays), among others). GFAT activity may be measured spectrophotometrically, using fructose-6-phosphate, glutamine, and 3-acetylpyridine adenine dinucleotide as substrates. Here, the change of absorbance resulting from reduction of 3-acetylpyridine adenine dinucleotide is monitored spectrophotometrically (McKnight, G. L et al (1992) J. Biol. Chem. 267: 25208-25212; Traxinger, R. R., and Marshall, S. (1991) J. Biol. Chem. 266:10148-10154).

**Apoptosis assays.** Apoptosis or programmed cell death is a suicide program is activated within the cell, leading to fragmentation of DNA, shrinkage of the cytoplasm, membrane changes and cell death. Apoptosis is mediated by proteolytic enzymes of the caspase family. Many of the altering parameters of a cell are measurable during apoptosis. Assays for apoptosis may be performed by terminal deoxynucleotidyl transferase-mediated digoxigenin-11-dUTP nick end labeling (TUNEL) assay. The TUNEL assay is used to measure nuclear DNA fragmentation characteristic of apoptosis (Lazebnik et al., 1994, Nature 371, 346), by following the incorporation of fluorescein-dUTP (Yonehara et al., 1989, J. Exp. Med. 169, 1747). Apoptosis may further be assayed by acridine orange staining of tissue culture cells (Lucas, R., et al., 1998, Blood 15:4730-41). Other cell-based apoptosis assays include the caspase-3/7 assay and the cell death nucleosome ELISA assay. The caspase 3/7 assay is based on the activation of the caspase cleavage activity as part of a cascade of events that occur during programmed cell death in many apoptotic pathways. In the caspase 3/7 assay (commercially available Apo-ONE^{™} Homogeneous Caspase-3/7 assay from Promega, cat# 67790), lysis buffer and caspase substrate are mixed and added to cells. The caspase substrate becomes fluorescent when cleaved by active caspase 3/7. The nucleosome ELISA assay is a general cell death assay known to those skilled in the art, and available commercially (Roche, Cat# 1774425). This assay is a quantitative sandwich-enzyme-immunoassay which uses monoclonal antibodies directed against DNA and histones respectively, thus specifically determining amount of mono- and oligonucleosomes in the cytoplasmic fraction of cell lysates. Mono and oligonucleosomes are enriched in the cytoplasm during apoptosis due to the fact that DNA fragmentation occurs several hours before the plasma membrane breaks down, allowing for accumalation in the cytoplasm. Nucleosomes are not present in the cytoplasmic fraction of cells that are not undergoing apoptosis. The Phospho-histone H2B assay is another apoptosis assay, based on phosphorylation of histone H2B as a result of apoptosis. Fluorescent dyes that are associated with phosphohistone H2B may be used to measure the increase of phosphohistone H2B as a result of apoptosis. Apoptosis assays that simultaneously measure multiple parameters associated with apoptosis have also been developed. In such assays, various cellular parameters that can be associated with antibodies or fluorescent dyes, and that mark various stages of apoptosis are labeled, and the results are measured using instruments such as Cellomics^{™} ArrayScan^{®} HCS System. The measurable parameters and their markers include anti-active caspase-3 antibody which marks intermediate stage apoptosis, anti-PARP-p85 antibody (cleaved PARP) which marks late stage apoptosis, Hoechst labels which label the nucleus and are used to measure nuclear swelling as a measure of early apoptosis and nuclear condensation as a measure of late apoptosis, TOTO-3 fluorescent dye which labels DNA of dead cells with high cell membrane permeability, and anti-alpha-tubulin or F-actin labels, which assess cytoskeletal changes in cells and correlate well with TOTO-3 label..

An apoptosis assay system may comprise a cell that expresses a GFAT, and that optionally has defective Axin function (e.g. Axin is over-expressed or under-expressed relative to wild-type cells). A test agent can be added to the apoptosis assay system and changes in induction of apoptosis relative to controls where no test agent is added, identify candidate Axin modulating agents. In some embodiments of the invention, an apoptosis assay may be used as a secondary assay to test a candidate Axin modulating agents that is initially identified using a cell-free assay system. An apoptosis assay may also be used to test whether GFAT function plays a direct role in apoptosis. For example, an apoptosis assay may be performed on cells that over- or under-express GFAT relative to wild type cells. Differences in apoptotic response compared to wild type cells suggests that the GFAT plays a direct role in the apoptotic response. Apoptosis assays are described further in US Pat. No. 6,133,437.

**Cell proliferation and cell cycle assays.** Cell proliferation may be assayed via bromodeoxyuridine (BRDU) incorporation. This assay identifies a cell population undergoing DNA synthesis by incorporation of BRDU into newly-synthesized DNA. Newly-synthesized DNA may then be detected using an anti-BRDU antibody (Hoshino et al., 1986, Int. J. Cancer 38, 369; Campana et al., 1988, J. Immunol. Meth. 107, 79), or by other means.

Cell proliferation is also assayed via phospho-histone H3 staining, which identifies a cell population undergoing mitosis by phosphorylation of histone H3. Phosphorylation of histone H3 at serine 10 is detected using an antibody specfic to the phosphorylated form of the serine 10 residue of histone H3. (Chadlee,D.N. 1995, J. Biol. Chem 270:20098-105). Cell Proliferation may also be examined using [³H]-thymidine incorporation (Chen, J., 1996, Oncogene 13:1395-403; Jeoung, J., 1995, J. Biol. Chem. 270:18367-73). This assay allows for quantitative characterization of S-phase DNA syntheses. In this assay, cells synthesizing DNA will incorporate [³H]-thymidine into newly synthesized DNA. Incorporation can then be measured by standard techniques such as by counting of radioisotope in a scintillation counter (e.g., Beckman LS 3800 Liquid Scintillation Counter). Another proliferation assay uses the dye Alamar Blue (available from Biosource International), which fluoresces when reduced in living cells and provides an indirect measurement of cell number (Voytik-Harbin SL et al., 1998, In Vitro Cell Dev Biol Anim 34:239-46). Yet another proliferation assay, the MTS assay, is based on in vitro cytotoxicity assessment of industrial chemicals, and uses the soluble tetrazolium salt, MTS. MTS assays are commercially available, for example, the Promega CellTiter 96^{®} AQueous NonRadioactive Cell Proliferation Assay (Cat.# G5421).

Cell proliferation may also be assayed by colony formation in soft agar, or clonogenic survival assay (Sambrook et al., Molecular Cloning, Cold Spring Harbor (1989)). For example, cells transformed with GFAT are seeded in soft agar plates, and colonies are measured and counted after two weeks incubation.

Cell proliferation may also be assayed by measuring ATP levels as indicator of metabolically active cells. Such assays are commercially available, for example Cell Titer-Glo™, which is a luminescent homogeneous assay available from Promega.

Involvement of a gene in the cell cycle may be assayed by flow cytometry (Gray JW et al. (1986) Int J Radiat Biol Relat Stud Phys Chem Med 49:237-55). Cells transfected with a GFAT may be stained with propidium iodide and evaluated in a flow cytometer (available from Becton Dickinson), which indicates accumulation of cells in different stages of the cell cycle.

Accordingly, a cell proliferation or cell cycle assay system may comprise a cell that expresses a GFAT, and that optionally has defective Axin function (e.g. Axin is over-expressed or under-expressed relative to wild-type cells). A test agent can be added to the assay system and changes in cell proliferation or cell cycle relative to controls where no test agent is added, identify candidate Axin modulating agents. In some embodiments of the invention, the cell proliferation or cell cycle assay may be used as a secondary assay to test a candidate Axin modulating agents that is initially identified using another assay system such as a cell-free assay system. A cell proliferation assay may also be used to test whether GFAT function plays a direct role in cell proliferation or cell cycle. For example, a cell proliferation or cell cycle assay may be performed on cells that over- or under-express GFAT relative to wild type cells. Differences in proliferation or cell cycle compared to wild type cells suggests that the GFAT plays a direct role in cell proliferation or cell cycle.

**Angiogenesis.** Angiogenesis may be assayed using various human endothelial cell systems, such as umbilical vein, coronary artery, or dermal cells. Suitable assays include Alamar Blue based assays (available from Biosource International) to measure proliferation; migration assays using fluorescent molecules, such as the use of Becton Dickinson Falcon HTS FluoroBlock cell culture inserts to measure migration of cells through membranes in presence or absence of angiogenesis enhancer or suppressors; and tubule formation assays based on the formation of tubular structures by endothelial cells on Matrigel® (Becton Dickinson). Accordingly, an angiogenesis assay system may comprise a cell that expresses a GFAT, and that optionally has defective Axin function (e.g. Axin is over-expressed or under-expressed relative to wild-type cells). A test agent can be added to the angiogenesis assay system and changes in angiogenesis relative to controls where no test agent is added, identify candidate Axin modulating agents. In some embodiments of the invention, the angiogenesis assay may be used as a secondary assay to test a candidate Axin modulating agents that is initially identified using another assay system. An angiogenesis assay may also be used to test whether GFAT function plays a direct role in cell proliferation. For example, an angiogenesis assay may be performed on cells that over- or under-express GFAT relative to wild type cells. Differences in angiogenesis compared to wild type cells suggests that the GFAT plays a direct role in angiogenesis. U.S. Pat. Nos. 5,976,782, 6,225,118 and 6,444,434, among others, describe various angiogenesis assays.

**Hypoxic induction**. The alpha subunit of the transcription factor, hypoxia inducible factor-1 (HIF-1), is upregulated in tumor cells following exposure to hypoxia in vitro. Under hypoxic conditions, HIF-1 stimulates the expression of genes known to be important in tumour cell survival, such as those encoding glyolytic enzymes and VEGF. Induction of such genes by hypoxic conditions may be assayed by growing cells transfected with GFAT in hypoxic conditions (such as with 0.1 % 02, 5% CO2, and balance N2, generated in a Napco 7001 incubator (Precision Scientific)) and normoxic conditions, followed by assessment of gene activity or expression by Taqman®. For example, a hypoxic induction assay system may comprise a cell that expresses a GFAT, and that optionally has defective Axin function (e.g. Axin is over-expressed or under-expressed relative to wild-type cells). A test agent can be added to the hypoxic induction assay system and changes in hypoxic response relative to controls where no test agent is added, identify candidate Axin modulating agents. In some embodiments of the invention, the hypoxic induction assay may be used as a secondary assay to test a candidate Axin modulating agents that is initially identified using another assay system. A hypoxic induction assay may also be used to test whether GFAT function plays a direct role in the hypoxic response. For example, a hypoxic induction assay may be performed on cells that over- or under-express GFAT relative to wild type cells. Differences in hypoxic response compared to wild type cells suggests that the GFAT plays a direct role in hypoxic induction.

**Cell adhesion**. Cell adhesion assays measure adhesion of cells to purified adhesion proteins, or adhesion of cells to each other, in presence or absence of candidate modulating agents. Cell-protein adhesion assays measure the ability of agents to modulate the adhesion of cells to purified proteins. For example, recombinant proteins are produced, diluted to 2.5g/mL in PBS, and used to coat the wells of a microtiter plate. The wells used for negative control are not coated. Coated wells are then washed, blocked with 1% BSA, and washed again. Compounds are diluted to 2× final test concentration and added to the blocked, coated wells. Cells are then added to the wells, and the unbound cells are washed off. Retained cells are labeled directly on the plate by adding a membrane-permeable fluorescent dye, such as calcein-AM, and the signal is quantified in a fluorescent microplate reader.

Cell-cell adhesion assays measure the ability of agents to modulate binding of cell adhesion proteins with their native ligands. These assays use cells that naturally or recombinantly express the adhesion protein of choice. In an exemplary assay, cells expressing the cell adhesion protein are plated in wells of a multiwell plate. Cells expressing the ligand are labeled with a membrane-permeable fluorescent dye, such as BCECF , and allowed to adhere to the monolayers in the presence of candidate agents. Unbound cells are washed off, and bound cells are detected using a fluorescence plate reader.

High-throughput cell adhesion assays have also been described. In one such assay, small molecule ligands and peptides are bound to the surface of microscope slides using a microarray spotter, intact cells are then contacted with the slides, and unbound cells are washed off. In this assay, not only the binding specificity of the peptides and modulators against cell lines are determined, but also the functional cell signaling of attached cells using immunofluorescence techniques in situ on the microchip is measured (Falsey JR et al., Bioconjug Chem. 2001 May-Jun;12(3):346-53).

### Primary assays for antibody modulators,

For antibody modulators, appropriate primary assays test is a binding assay that tests the antibody's affinity to and specificity for the GFAT protein. Methods for testing antibody affinity and specificity are well known in the art (Harlow and Lane, 1988, 1999, *supra).* The enzyme-linked immunosorbant assay (ELISA) is a preferred method for detecting GFAT-specific antibodies; others include FACS assays, radioimmunoassays, and fluorescent assays.

In some cases, screening assays described for small molecule modulators may also be used to test antibody modulators.

### Primary assays for nucleic acid modulators

For nucleic acid modulators, primary assays may test the ability of the nucleic acid modulator to inhibit or enhance GFAT gene expression, preferably mRNA expression. In general, expression analysis comprises comparing GFAT expression in like populations of cells (*e.g*., two pools of cells that endogenously or recombinantly express GFAT) in the presence and absence of the nucleic acid modulator. Methods for analyzing mRNA and protein expression are well known in the art. For instance, Northern blotting, slot blotting, ribonuclease protection, quantitative RT-PCR (*e.g*., using the TaqMan®, PE Applied Biosystems), or microarray analysis may be used to confirm that GFAT mRNA expression is reduced in cells treated with the nucleic acid modulator (*e.g*., Current Protocols in Molecular Biology (1994) Ausubel FM et al., eds., John Wiley & Sons, Inc., chapter 4; Freeman WM et al., Biotechniques (1999) 26:112-125; Kallioniemi OP, Ann Med 2001, 33:142-147; Blohm DH and Guiseppi-Elie, A Curr Opin Biotechnol 2001, 12:41-47). Protein expression may also be monitored. Proteins are most commonly detected with specific antibodies or antisera directed against either the GFAT protein or specific peptides. A variety of means including Western blotting, ELISA, or in situ detection, are available (Harlow E and Lane D, 1988 and 1999, *supra*).

In some cases, screening assays described for small molecule modulators, particularly in assay systems that involve GFAT mRNA expression, may also be used to test nucleic acid modulators.

### Secondary Assays

Secondary assays may be used to further assess the activity of GFAT-modulating agent identified by any of the above methods to confirm that the modulating agent affects GFAT in a manner relevant to the Axin pathway. As used herein, GFAT-modulating agents encompass candidate clinical compounds or other agents derived from previously identified modulating agent. Secondary assays can also be used to test the activity of a modulating agent on a particular genetic or biochemical pathway or to test the specificity of the modulating agent's interaction with GFAT.

Secondary assays generally compare like populations of cells (*e.g*., two pools of cells that endogenously or recombinantly express GFAT) in the presence and absence of the candidate modulator. In general, such assays test whether treatment of cells with a candidate GFAT-modulating agent results in changes in the Axin pathway in comparison to untreated (or mock- or placebo-treated) cells or animals. Certain assays use "sensitized genetic backgrounds", which, as used herein, describe cells or animals engineered for altered expression of genes in the Axin or interacting pathways.

### Cell-based assays

Cell based assays may detect endogenous Axin pathway activity or may rely on recombinant expression of Axin pathway components. Any of the aforementioned assays may be used in this cell-based format. Candidate modulators are typically added to the cell media but may also be injected into cells or delivered by any other efficacious means.

### Animal Assays (not part of the invention)

A variety of non-human animal models of normal or defective Axin pathway may be used to test candidate GFAT modulators. Models for defective Axin pathway typically use genetically modified animals that have been engineered to mis-express (*e.g*., over-express or lack expression in) genes involved in the Axin pathway. Assays generally require systemic delivery of the candidate modulators, such as by oral administration, injection, etc.

Axin pathway activity may be assessed by monitoring neovascularization and angiogenesis. Animal models with defective and normal Axin are used to test the candidate modulator's affect on GFAT in Matrigel® assays. Matrigel® is an extract of basement membrane proteins, and is composed primarily of laminin, collagen IV, and heparin sulfate proteoglycan. It is provided as a sterile liquid at 4° C, but rapidly forms a solid gel at 37° C. Liquid Matrigel® is mixed with various angiogenic agents, such as bFGF and VEGF, or with human tumor cells which over-express the GFAT. The mixture is then injected subcutaneously(SC) into female athymic nude mice (Taconic, Germantown, NY) to support an intense vascular response. Mice with Matrigel® pellets may be dosed via oral (PO), intraperitoneal (IP), or intravenous (IV) routes with the candidate modulator. Mice are euthanized 5 - 12 days post-injection, and the Matrigel® pellet is harvested for hemoglobin analysis (Sigma plasma hemoglobin kit). Hemoglobin content of the gel is found to correlate the degree of neovascularization in the gel.

The effect of the candidate modulator on GFAT may be assessed via tumorigenicity assays. Tumor xenograft assays are known in the art (see, e.g., Ogawa K et al., 2000, Oncogene 19:6043-6052). Xenografts are typically implanted SC into female athymic mice, 6-7 week old, as single cell suspensions either from a pre-existing tumor or from *in vitro* culture. The tumors which express the GFAT endogenously are injected in the flank, 1 x 10⁵ to 1 x 10⁷ cells per mouse in a volume of 100 µL using a 27 gauge needle. Mice are then ear tagged and tumors are measured twice weekly. Candidate modulator treatment is initiated on the day the mean tumor weight reaches 100 mg. Candidate modulator is delivered IV, SC, IP, or PO by bolus administration. Depending upon the pharmacokinetics of each unique candidate modulator, dosing can be performed multiple times per day. The tumor weight is assessed by measuring perpendicular diameters with a caliper and calculated by multiplying the measurements of diameters in two dimensions. At the end of the experiment, the excised tumors maybe utilized for biomarker identification or further analyses. For immunohistochemistry staining, xenograft tumors are fixed in 4% paraformaldehyde, 0.1M phosphate, pH 7.2, for 6 hours at 4°C, immersed in 30% sucrose in PBS, and rapidly frozen in isopentane cooled with liquid nitrogen.

Tumorogenicity may be monitored using a hollow fiber assay, which is described in U.S. Pat No. US 5,698,413. Briefly, the method comprises implanting into a laboratory animal a biocompatible, semi-permeable encapsulation device containing target cells, treating the laboratory animal with a candidate modulating agent, and evaluating the target cells for reaction to the candidate modulator. Implanted cells are generally human cells from a pre-existing tumor or a tumor cell line. After an appropriate period of time, generally around six days, the implanted samples are harvested for evaluation of the candidate modulator. Tumorogenicity and modulator efficacy may be evaluated by assaying the quantity of viable cells present in the macrocapsule, which can be determined by tests known in the art, for example, MTT dye conversion assay, neutral red dye uptake, trypan blue staining, viable cell counts, the number of colonies formed in soft agar, the capacity of the cells to recover and replicate in vitro, etc.

A tumorogenicity assay may use a transgenic animal, usually a mouse, carrying a dominant oncogene or tumor suppressor gene knockout under the control of tissue specific regulatory sequences; these assays are generally referred to as transgenic tumor assays. In a preferred application, tumor development in the transgenic model is well characterized or is controlled. In an exemplary model, the "RIP1-Tag2" transgene, comprising the SV40 large T-antigen oncogene under control of the insulin gene regulatory regions is expressed in pancreatic beta cells and results in islet cell carcinomas (Hanahan D, 1985, Nature 315:115-122; Parangi S et al, 1996, Proc Natl Acad Sci USA 93: 2002-2007; Bergers G et al, 1999, Science 284:808-812). An "angiogenic switch," occurs at approximately five weeks, as normally quiescent capillaries in a subset of hyperproliferative islets become angiogenic. The RIP1-TAG2 mice die by age 14 weeks. Candidate modulators may be administered at a variety of stages, including just prior to the angiogenic switch (e.g., for a model of tumor prevention), during the growth of small tumors (e.g., for a model of intervention), or during the growth of large and/or invasive tumors (e.g., for a model of regression). Tumorogenicity and modulator efficacy can be evaluating life-span extension and/or tumor characteristics, including number of tumors, tumor size, tumor morphology, vessel density, apoptotic index, etc.

### Diagnostic and therapeutic uses

Specific GFAT-modulating agents are useful in a variety of diagnostic and therapeutic applications where disease or disease prognosis is related to defects in the Axin pathway, such as angiogenic, apoptotic, or cell proliferation disorders. Accordingly, the invention also provides in vitro methods for modulating the Axin pathway in a cell, preferably a cell pre-determined to have defective or impaired Axin function (e.g. due to overexpression, underexpression, or misexpression of Axin, or due to gene mutations), comprising the step of administering an agent to the cell that specifically modulates GFAT activity. Preferably, the modulating agent produces a detectable phenotypic change in the cell indicating that the Axin function is restored. The phrase "function is restored", and equivalents, as used herein, means that the desired phenotype is achieved, or is brought closer to normal compared to untreated cells. For example, with restored Axin function, cell proliferation and/or progression through cell cycle may normalize, or be brought closer to normal relative to untreated cells. The invention also discloses methods for treating disorders or disease associated with impaired Axin function by administering a therapeutically effective amount of a GFAT -modulating agent that modulates the Axin pathway. The invention further discloses methods for modulating GFAT function in a cell, preferably a cell pre-determined to have defective or impaired GFAT function, by administering a GFAT -modulating agent. Additionally, the invention provides a method for treating disorders or disease associated with impaired GFAT function by administering a therapeutically effective amount of a GFAT -modulating agent.

The discovery that GFAT is implicated in Axin pathway provides for a variety of methods that can be employed for the diagnostic and prognostic evaluation of diseases and disorders involving defects in the Axin pathway and for the identification of subjects having a predisposition to such diseases and disorders.

Various expression analysis methods can be used to diagnose whether GFAT expression occurs in a particular sample, including Northern blotting, slot blotting, ribonuclease protection, quantitative RT-PCR, and microarray analysis. (*e.g*., Current Protocols in Molecular Biology (1994) Ausubel FM et al., eds., John Wiley & Sons, Inc., chapter 4; Freeman WM et al., Biotechniques (1999) 26:112-125; Kallioniemi OP, Ann Med 2001, 33:142-147; Blohm and Guiseppi-Elie, Curr Opin Biotechnol 2001, 12:41-47). Tissues having a disease or disorder implicating defective Axin signaling that express a GFAT, are identified as amenable to treatment with a GFAT modulating agent. In a preferred application, the Axin defective tissue overexpresses a GFAT relative to normal tissue. For example, a Northern blot analysis of mRNA from tumor and normal cell lines, or from tumor and matching normal tissue samples from the same patient, using full or partial GFAT cDNA sequences as probes, can determine whether particular tumors express or overexpress GFAT. Alternatively, the TaqMan® is used for quantitative RT-PCR analysis of GFAT expression in cell lines, normal tissues and tumor samples (PE Applied Biosystems).

Various other diagnostic methods may be performed, for example, utilizing reagents such as the GFAT oligonucleotides, and antibodies directed against a GFAT, as described above for: (1) the detection of the presence of GFAT gene mutations, or the detection of either over- or under-expression of GFAT mRNA relative to the non-disorder state; (2) the detection of either an over- or an under-abundance of GFAT gene product relative to the non-disorder state; and (3) the detection of perturbations or abnormalities in the signal transduction pathway mediated by GFAT.

Kits for detecting expression of GFAT in various samples, comprising at least one antibody specific to GFAT, all reagents and/or devices suitable for the detection of antibodies, the immobilization of antibodies, and the like, and instructions for using such kits in diagnosis or therapy are also disclosed.

Thus, in a specific embodiment, the invention is drawn to a method for diagnosing a disease or disorder in a patient that is associated with alterations in GFAT expression as defined by claims 17-18.

### EXAMPLES

The following experimental section and examples are offered by way of illustration and not by way of limitation.

### I. C. elegans Axin screen

We have found that the temperature-sensitive, reduction-of function pry-1 mutant mu38 grown at 15oC produces a ruptured vulva (Rvl) phenotype by which about 95% of animals become eviscerated and die at the L4 molt. The pry-1 Rvl mutant phenotype is suppressed by loss-of-function mutations in the beta-catenin ortholog bar-1 and the TCF ortholog pop-1. The Rvl phenotype can also be generated by gain-of function mutations in bar-1/beta-catenin that eliminate the consensus GSK3-beta phosphorylation sites and are predicted to prevent Axin-mediated degradation of BAR-1.

We designed a genetic screen to identify genes in addition to bar-1/beta-catenin and pop-1/TCF that act positively in beta-catenin signaling and, when inactivated, suppress the Rvl mutant phenotype of pry-1/Axin. The function of individual genes was inactivated by RNAi in pry-1 (mu38) L1 larvae, and suppression of the Rvl phenotype was scored as a statistically significant increase in the proportion of larvae that survived to adulthood without rupturing. Suppressor genes were subsequently counterscreened to eliminate those that appeared to suppress the pry-1 mutant non-specifically, rather than those that specifically functioned in beta-catenin signaling. Suppressor genes that did not block vulva formation in a wildtype background, and that did not suppress the Rvl phenotype of two mutations in genes unrelated to beta-catenin signaling (lin-1/Ets and daf-18/PTEN) were considered to be specific pry-1/Axin suppressors. These suppressor genes, when inactivated, likely suppress beta-catenin's inappropriate transcriptional activation of target genes and, therefore, may be relevant for cancer therapy. F07A11.2 was identified as a suppressor in the assay. Orthologs of F07A11.2 are referred to herein as GFAT.

BLAST analysis (Altschul et al., *supra)* was employed to identify orthologs of F07A11.2. For example, representative sequences from GFAT, GI# 4503981 (SEQ ID NO:7), and GI#4826742 (SEQ ID NO:8) share 60% and 61% amino acid identity, respectively, with the *C.elegans* F07A11.2.

Various domains, signals, and functional subunits in proteins were analyzed using the PSORT (Nakai K., and Horton P., Trends Biochem Sci, 1999, 24:34-6; Kenta Nakai, Protein sorting signals and prediction of subcellular localization, Adv. Protein Chem. 54,277-344 (2000)), PFAM (Bateman A., et al., Nucleic Acids Res, 1999, 27:260-2), SMART (Ponting CP, et al., SMART: identification and annotation of domains from signaling and extracellular protein sequences. Nucleic Acids Res. 1999 Jan 1;27(1):229-32), TM-HMM (Erik L.L. Sonnhammer, Gunnar von Heijne, and Anders Krogh: A hidden Markov model for predicting transmembrane helices in protein sequences. In Proc. of Sixth Int. Conf. on Intelligent Systems for Molecular Biology, p 175-182 Ed J. Glasgow, T. Littlejohn, F. Major, R. Lathrop, D. Sankoff, and C. Sensen Menlo Park, CA: AAAI Press, 1998), and clust (Remm M, and Sonnhammer E. Classification of transmembrane protein families in the Caenorhabditis elegans genome and identification of human orthologs. Genome Res. 2000 Nov;10(11):1679-89) programs. For example, the Glutamine amidotransferases class-II domains(PFAM 00310) of GFATs from GI#s 4503981 (SEQ ID NO:7) and 4826742 (SEQ ID NO:8) are located at approximately amino acid residues 2-263 and 2-155, respectively. Likewise, the SIS domain (PFAM01380) of GFATs from GI#s 4503981 (SEQ ID NO:7) and 4826742 (SEQ ID NO:8) are located at approximately amino acid residues 360 to 494, 531 to 667 for SEQ ID NO:7 and 361 to 495, 532 to 668 for SEQ ID NO:8, respectively.

### II. High-Throughput In Vitro Fluorescence Polarization Assay

Fluorescently-labeled GFAT peptide/substrate are added to each well of a 96-well microtiter plate, along with a test agent in a test buffer (10 mM HEPES, 10 mM NaCl, 6 mM magnesium chloride, pH 7.6). Changes in fluorescence polarization, determined by using a Fluorolite FPM-2 Fluorescence Polarization Microtiter System (Dynatech Laboratories, Inc), relative to control values indicates the test compound is a candidate modifier of GFAT activity.

### III. High-Throughput In Vitro Binding Assay.

³³P-labeled GFAT peptide is added in an assay buffer (100 mM KCl, 20 mM HEPES pH 7.6, 1 mM MgCl₂, 1% glycerol, 0.5% NP-40, 50 mM betamercaptoethanol, 1 mg/ml BSA, cocktail of protease inhibitors) along with a test agent to the wells of a Neutralite-avidin coated assay plate and incubated at 25°C for 1 hour. Biotinylated substrate is then added to each well and incubated for 1 hour. Reactions are stopped by washing with PBS, and counted in a scintillation counter. Test agents that cause a difference in activity relative to control without test agent are identified as candidate Axin modulating agents.

### IV. Immunoprecipitations and Immunoblotting

For coprecipitation of transfected proteins, 3 × 10⁶ appropriate recombinant cells containing the GFAT-proteins are plated on 10-cm dishes and transfected on the following day with expression constructs. The total amount of DNA is kept constant in each transfection by adding empty vector. After 24 h, cells are collected, washed once with phosphate-buffered saline and lysed for 20 min on ice in 1 ml of lysis buffer containing 50 mM Hepes, pH 7.9, 250 mM NaCl, 20 mM -glycerophosphate, 1 mM sodium orthovanadate, 5 mM p-nitrophenyl phosphate, 2 mM dithiothreitol, protease inhibitors (complete, Roche Molecular Biochemicals), and 1% Nonidet P-40. Cellular debris is removed by centrifugation twice at 15,000 × g for 15 min. The cell lysate is incubated with 25 µl of M2 beads (Sigma) for 2 h at 4 °C with gentle rocking.

After extensive washing with lysis buffer, proteins bound to the beads are solubilized by boiling in SDS sample buffer, fractionated by SDS-polyacrylamide gel electrophoresis, transferred to polyvinylidene difluoride membrane and blotted with the indicated antibodies. The reactive bands are visualized with horseradish peroxidase coupled to the appropriate secondary antibodies and the enhanced chemiluminescence (ECL) Western blotting detection system (Amersham Pharmacia Biotech).

### V. Expression analysis

All cell lines used in the following experiments are NCI (National Cancer Institute) lines, and are available from ATCC (American Type Culture Collection, Manassas, VA 20110-2209). Normal and tumor tissues were obtained from Impath, UC Davis, Clontech, Stratagene, Ardais, Genome Collaborative, and Ambion.

TaqMan^{®} analysis was used to assess expression levels of the disclosed genes in various samples.

RNA was extracted from each tissue sample using Qiagen (Valencia, CA) RNeasy kits, following manufacturer's protocols, to a final concentration of 50ng/µl. Single stranded cDNA was then synthesized by reverse transcribing the RNA samples using random hexamers and 500ng of total RNA per reaction, following protocol 4304965 of Applied Biosystems (Foster City, CA).

Primers for expression analysis using TaqMan® assay (Applied Biosystems, Foster City, CA) were prepared according to the TaqMan® protocols, and the following criteria: a) primer pairs were designed to span introns to eliminate genomic contamination, and b) each primer pair produced only one product. Expression analysis was performed using a 7900HT instrument.

TaqMan® reactions were carried out following manufacturer's protocols, in 25 µl total volume for 96-well plates and 10 µl total volume for 384-well plates, using 300nM primer and 250 nM probe, and approximately 25ng of cDNA. The standard curve for result analysis was prepared using a universal pool of human cDNA samples, which is a mixture of cDNAs from a wide variety of tissues so that the chance that a target will be present in appreciable amounts is good. The raw data were normalized using 18S rRNA (universally expressed in all tissues and cells).

For each expression analysis, tumor tissue samples were compared with matched normal tissues from the same patient. A gene was considered overexpressed in a tumor when the level of expression of the gene was 2 fold or higher in the tumor compared with its matched normal sample. In cases where normal tissue was not available, a universal pool of cDNA samples was used instead. In these cases, a gene was considered overexpressed in a tumor sample when the difference of expression levels between a tumor sample and the average of all normal samples from the same tissue type was greater than 2 times the standard deviation of all normal samples (i.e., Tumor - average(all normal samples) > 2 x STDEV(all normal samples)).

Results are shown in Table 1. Number of pairs of tumor samples and matched normal tissue from the same patient are shown for each tumor type. Percentage of the samples with at least two-fold overexpression for each tumor type is provided. A modulator identified by an assay described herein can be further validated for therapeutic effect by administration to a tumor in which the gene is overexpressed. A decrease in tumor growth confirms therapeutic utility of the modulator. Prior to treating a patient with the modulator, the likelihood that the patient will respond to treatment can be diagnosed by obtaining a tumor sample from the patient, and assaying for expression of the gene targeted by the modulator. The expression data for the gene(s) can also be used as a diagnostic marker for disease progression. The assay can be performed by expression analysis as described above, by antibody directed to the gene target, or by any other available detection method.

**Table 1**

| SEQ ID NO | 2 | 3 |
|---|---|---|
| Breast | 10% | 10% |
| # of Pairs | 21 | 20 |
| Colon | 21% | 36% |
| # of Pairs | 33 | 33 |
| Head And Neck | 0% | 25% |
| # of Pairs | 8 | 8 |
| Kidney | 12% | 25% |
| # of Pairs | 24 | 24 |
| Lung | 23% | 18% |
| # of Pairs | 22 | 22 |
| Ovary | 33% | 0% |
| # of Pairs | 12 | 12 |
| Prostate | 17% | 8% |
| # of Pairs | 12 | 12 |
| Skin | 33% | 33% |
| # of Pairs | 3 | 3 |
| Uterus | 21% | 0% |
| # of Pairs | 19 | 18 |

### VI. GFAT functional assays

We used the transferase assays described above to assess whether GFAT behaves as a transferase. Our results indicate that GFAT SEQID numbers 6, 7, and 8 are transferase enzymes.

RNAi experiments were then carried out to knock down expression of GFAT (SEQ ID Nos: 2 and 3) in various cell lines using small interfering RNAs (siRNA, Elbashir et al, *supra*).

**Effect of GFAT RNAi on cell proliferation and growth**. BrdU and Cell Titer-Glo™ assays, as described above, were employed to study the effects of decreased GFAT expression on cell proliferation. The results of these experiments indicated that RNAi of SEQ ID NO: 2 decreases proliferation in LX1 lung cancer cells and 231T breast cancer cells.

MTS cell proliferation assay, as described above, was also employed to study the effects of decreased GFAT expression on cell proliferation. The results of this experiment indicated that RNAi of SEQ ID NO:2 decreased proliferation in LX1 and A549 lung cancer cells, HCT116 colon cancer cells, LNCAP prostate cancer cells, and SKBR3 breast cancer cells.

**Effect of GFAT RNAi on apoptosis**. Nucleosome ELISA apoptosis assay, as described above, was employed to study the effects of decreased GFAT expression on apoptosis. The results of this experiment indicated that RNAi of GFAT of SEQ ID NO:2 increased apoptosis in LX1 lung cancer cells.

PARP cleavage assay was also used to assess the effects of decreased GFAT expression on apoptosis. In this assay, antibodies were produced against PARP, which is a protein with increased levels in apoptosis. Levels of PARP were measured in a Western blot as a result of GFAT knockdown in various cell lines. The result of this experiment indicated that RNAi of SEQ ID NO:2 increased PARP levels in 231T breast cancer and A549 lung cancer cells.

**High Throughput Beta Catenin Transcriptional readout assay**. This assay is an expanded TaqMan® transcriptional readout assay monitoring changes in the mRNA levels of endogenous beta catenin regulated genes. This assay measures changes in expression of beta catenin regulated cellular genes as a readout for axin/beta catenin pathway signaling activity.

We identified a panel of genes that were transcriptionally regulated by beta catenin signaling, then designed and tested TaqMan® primer/probes sets. We reduced expression of beta catenin by RNAi, and tested its affect on the expression of the transcriptionally regulated genes in multiple cell types. The panel readout was then narrowed to the ten most robust probes.

We then treated cancer cells with siRNAs of the target genes of interest, such as GFAT, and tested how the reduced levels of the target genes affected the expression levels of the beta catenin regulated gene panel.

Genes that when knocked out via RNAi, demonstrated the same pattern of activity on at least one panel gene as a beta-catenin knockout, were identified as involved in the beta catenin pathway.

TaqMan® assays were performed on the RNAs in a 384 well format.

RNAi of both SEQ ID Nos: 2 and 3 showed the same pattern of activity as beta catenin RNAi for at least one of the transcriptionally regulated genes. This data suggests a direct relationship of GFAT with the Axin/beta catenin pathways.

### VII. GFAT IHC

Immunohistochemistry was used to localize GFAT protein in human tissue sections according to known methods (Thomas Boenisch, ed. (2001) Handbook, Immunochemical Staining Methods, 3rd Edition, Dako Corporation, Carpinteria, CA). Affinity purified rabbit polyclonal antibodies were produced against GFAT peptides. Also, crude mouse serum was produced against SEQID NO:7 and crude rabbit serum was produced against SEQ ID NO:8. All antibodies against SEQ ID NO:7 and its peptides worked well in both ELISA and Western blot assays. Antibodies were then tested in IHC in human cancer tissue samples, and compared with matched normal tissue from the same patient. Results of this experiment indicated that SEQ ID NO:7 was highly expressed in breast, colon, kidney, lung and ovarian cancer samples as compared with expression in the matched normal tissue counterparts.

## Claims

1. An in vitro method of identifying a candidate Axin pathway modulating agent, said method comprising the steps of:
(a) providing an assay system capable of detecting glutamine: fructose-6 phosphate amidotransferase (GFAT) expression and/or GFAT transferase activity comprising a GFAT polypeptide or nucleic acid;
(b) contacting the assay system with a test agent under conditions whereby, but for the presence of the test agent, the system provides a reference activity; and
(c) determining the expression of said GFAT nucleic acid and/or activity of said GFAT polypeptide in the assay system, wherein a change in said expression or activity between the presence and absence of said test agent identifies the test agent as a candidate Axin pathway modulating agent.

2. The method of Claim 1 wherein the assay system comprises cultured cells that express the GFAT polypeptide.

3. The method of Claim 2 wherein the cultured cells additionally have defective Axin function.

4. The method of Claim 1 wherein the assay system includes a screening assay comprising a GFAT polypeptide, and the candidate test agent is a small molecule modulator.

5. The method of Claim 4 wherein the assay is a transferase assay.

6. The method of Claim 1 wherein the assay system includes a binding assay comprising a GFAT polypeptide and the candidate test agent is an antibody.

7. The method of Claim 1 wherein the assay system includes an expression assay comprising a GFAT nucleic acid and the candidate test agent is a nucleic acid modulator.

8. The method of claim 7 wherein the nucleic acid modulator is an antisense oligomer.

9. The method of Claim 7 wherein the nucleic acid modulator is a PMO.

10. The method of Claim 1 additionally comprising:
(d) administering the candidate Axin pathway modulating agent identified in (c) to an in vitro model system comprising cells defective in Axin function and detecting a phenotypic change in the model system that indicates that the Axin function is restored.

11. An in vitro method of modulating an Axin pathway in a mammalian cell comprising contacting the cell with an agent selected from the group consisting of a GFAT-specific antibody, a GFAT antisense oligomer or a GFAT RNAi molecule.

12. The method of claim 11, wherein the cell is defective in Axin function and whereby Axin function is restored upon contact with said agent.

13. A GFAT-specific antibody, a GFAT antisense oligomer or a GFAT RNAi molecule for use in the treatment of colon or prostate cancer.

14. The GFAT-specific antibody, GFAT antisense oligomer or GFAT RNAi molecule for use in treatment of colon or prostate cancer of claim 13 which is to be administered to a vertebrate animal predetermined to have said cancer.

15. The method of Claim 1, comprising the additional steps of:
(d) providing a second assay system comprising cultured cells expressing GFAT, wherein the second assay can detect a test agent-biased change in the Axin pathway
(e) contacting the second assay system with the test agent of (b) or an agent derived therefrom under conditions whereby, but for the presence of the test agent or agent derived therefrom, the system provides a reference activity; and
(f) detecting an agent-biased activity of the second assay system, wherein a difference between the agent-biased activity and the reference activity of the second assay system confirms the test agent or agent derived therefrom as a candidate Axin pathway modulating agent.

16. The method of Claim 15 wherein the second assay system is selected from the group consisting of an apoptosis assay system, a cell proliferation assay system, an angiogenesis assay system, and a hypoxic induction assay system.

17. An in vitro method for diagnosing head and neck, kidney, lung, prostate, skin or uterine cancer in a patient comprising:
(a) contacting a biological sample from the patient with a probe for GFAT expression;
(b) determining GFAT expression from step (a) and comparing the results with a control; and
(c) determining whether the difference between GFAT expression from the patient sample and the control as determined in step (b) indicates a likelihood of head and neck, kidney, lung, prostate, skin or uterine cancer.

18. The method according to claim 17, wherein said cancer is head and neck, kidney, or skin cancer.

## Patentansprüche

1. Ein In-vitro-Verfahren zum Identifizieren eines den Axin-Weg modulierenden Wirkstoffkandidaten, wobei das Verfahren die folgenden Schritte beinhaltet:
(a) Bereitstellen eines Assaysystems, das in der Lage ist, Glutamin-Fructose-6-Phosphat-Amidotransferase (GFAT)-Expression und/oder GFAT-Transferase-Aktivität zu erkennen, das ein GFAT-Polypeptid oder eine GFAT-Nucleinsäure beinhaltet;
(b) In-Kontakt-Bringen des Assaysystems mit einem Testwirkstoff unter Bedingungen, unter denen, außer in der Gegenwart des Testwirkstoffs, das System eine Referenzaktivität bereitstellt; und
(c) Bestimmen der Expression der GFAT-Nucleinsäure und/oder der Aktivität des GFAT-Polypeptids in dem Assaysystem, wobei eine Änderung bei der Expression oder Aktivität zwischen dem Vorhandensein und der Abwesenheit des Testwirkstoffs den Testwirkstoff als einen den Axin-Weg modulierenden Wirkstoffkandidaten identifiziert.

2. Verfahren gemäß Anspruch 1, wobei das Assaysystem kultivierte Zellen beinhaltet, die das GFAT-Polypeptid exprimieren.

3. Verfahren gemäß Anspruch 2, wobei die kultivierten Zellen zusätzlich eine fehlerhafte Axin-Funktion aufweisen.

4. Verfahren gemäß Anspruch 1, wobei das Assaysystem einen Screening-Assay umfasst, der ein GFAT-Polypeptid beinhaltet, und der Testwirkstoffkandidat ein Modulator aus einem kleinen Molekül ist.

5. Verfahren gemäß Anspruch 4, wobei der Assay ein Transferase-Assay ist.

6. Verfahren gemäß Anspruch 1, wobei das Assaysystem einen Bindungs-Assay umfasst, der ein GFAT-Polypeptid beinhaltet, und der Testwirkstoffkandidat ein Antikörper ist.

7. Verfahren gemäß Anspruch 1, wobei das Assaysystem einen Expressions-Assay umfasst, der eine GFAT-Nucleinsäure beinhaltet, und der Testwirkstoffkandidat ein Modulator aus Nucleinsäure ist.

8. Verfahren gemäß Anspruch 7, wobei der Modulator aus Nucleinsäure ein Antisense-Oligomer ist.

9. Verfahren gemäß Anspruch 7, wobei der Modulator aus Nucleinsäure ein PMO ist.

10. Verfahren gemäß Anspruch 1, das zusätzlich Folgendes beinhaltet:
(d) Verabreichen des in (c) identifizierten, den Axin-Weg modulierenden Wirkstoffkandidaten an ein In-vitro-Modellsystem, das hinsichtlich der Axin-Funktion fehlerhafte Zellen beinhaltet, und Erkennen einer phänotypischen Änderung in dem Modellsystem, die anzeigt, dass die Axin-Funktion wiederhergestellt ist.

11. Ein In-vitro-Verfahren zum Modulieren eines Axin-Wegs in einer Säugetierzelle, das das In-Kontakt-Bringen der Zelle mit einem Wirkstoff beinhaltet, der aus der Gruppe, bestehend aus einem GFAT-spezifischen Antikörper, einem GFAT-Antisense-Oligomer oder einem GFAT-RNAi-Molekül, ausgewählt ist.

12. Verfahren gemäß Anspruch 11, wobei die Zelle hinsichtlich der Axin-Funktion fehlerhaft ist und wobei die Axin-Funktion bei Kontakt mit dem Wirkstoff wiederhergestellt wird.

13. Ein GFAT-spezifischer Antikörper, ein GFAT-Antisense-Oligomer oder ein GFAT-RNAi-Molekül zur Verwendung bei der Behandlung von Kolon- oder Prostatakrebs.

14. GFAT-spezifischer Antikörper, GFAT-Antisense-Oligomer oder GFAT-RNAi-Molekül zur Verwendung bei der Behandlung von Kolon- oder Prostatakrebs gemäß Anspruch 13 zur Verabreichung an ein Wirbeltier, bei dem zuvor diese Art von Krebs festgestellt wurde.

15. Verfahren gemäß Anspruch 1, das die folgenden zusätzlichen Schritte beinhaltet:
(d) Bereitstellen eines zweiten Assaysystems, das GFAT exprimierende kultivierte Zellen beinhaltet, wobei der zweite Assay eine von dem Testwirkstoff beeinflusste Veränderung bei dem Axin-Weg erkennen kann;
(e) In-Kontakt-Bringen des zweiten Assaysystems mit dem Testwirkstoff aus (b) oder einem davon abgeleiteten Wirkstoff unter Bedingungen, unter denen, außer in der Gegenwart des Testwirkstoffs oder des davon abgeleiteten Wirkstoffs, das System eine Referenzaktivität bereitstellt; und
(f) Erkennen einer von dem Wirkstoff beeinflussten Aktivität des zweiten Assaysystems,
wobei ein Unterschied zwischen der von dem Wirkstoff beeinflussten Aktivität und der Referenzaktivität des zweiten Assaysystems den Testwirkstoff oder den davon abgeleiteten Wirkstoff als einen den Axin-Weg modulierenden Wirkstoffkandidaten bestätigt.

16. Verfahren gemäß Anspruch 15, wobei das zweite Assaysystem aus der Gruppe, bestehend aus einem Apoptose-Assaysystem, einem Zellproliferations-Assaysystem, einem Angiogenese-Assaysystem und einem Hypoxieinduktions-Assaysystem, ausgewählt ist.

17. Ein In-Vitro-Verfahren zum Diagnostizieren von Krebs von Kopf und Hals, der Nieren, der Lunge, der Prostata, der Haut oder der Gebärmutter bei einem Patienten, das Folgendes beinhaltet:
(a) In-Kontakt-Bringen einer biologischen Probe von dem Patienten mit einer Sonde für die GFAT-Expression;
(b) Bestimmen der GFAT-Expression aus Schritt (a) und Vergleichen der Ergebnisse mit einer Kontrolle und
(c) Bestimmen, ob der Unterschied zwischen der GFAT-Expression von der Patientenprobe und der wie in Schritt b) bestimmten Kontrolle eine Wahrscheinlichkeit von Krebs von Kopf und Hals, den Nieren, der Lunge, der Prostata, der Haut oder der Gebärmutter anzeigt.

18. Verfahren gemäß Anspruch 17, wobei der Krebs Krebs von Kopf und Hals, den Nieren oder der Haut ist.

## Revendications

1. Une méthode in vitro pour identifier un agent de modulation de voie de l'Axine candidat, ladite méthode comprenant les étapes consistant à :
(a) fournir un système d'épreuve capable de détecter l'expression de la glutamine:fructose-6 phosphate amidotransférase (GFAT) et / ou l'activité transférase de GFAT comprenant un polypeptide ou un acide nucléique de GFAT;
(b) mettre en contact le système d'épreuve avec un agent de test dans des conditions dans lesquelles, n'était-ce la présence de l'agent de test, le système fournit une activité de référence ; et
(c) déterminer l'expression dudit acide nucléique de GFAT et / ou l'activité dudit polypeptide de GFAT dans le système d'épreuve, dans laquelle un changement dans ladite expression ou l'activité entre la présence et l'absence dudit agent de test identifie l'agent de test comme étant l'agent de modulation de voie de l'Axine candidat.

2. La méthode de la revendication 1 dans laquelle le système d'épreuve comprend des cellules cultivées qui expriment le polypeptide de GFAT.

3. La méthode de la revendication 2 dans laquelle les cellules cultivées ont de plus une fonction Axine défectueuse.

4. La méthode de la revendication 1 dans laquelle le système d'épreuve comporte une épreuve de criblage comprenant un polypeptide de GFAT, et l'agent de test candidat est un modulateur à petites molécules.

5. La méthode de la revendication 4 dans laquelle l'épreuve est une épreuve de transférase.

6. La méthode de la revendication 1 dans laquelle le système d'épreuve comporte une épreuve de liaison comprenant un polypeptide de GFAT et l'agent de test candidat est un anticorps.

7. La méthode de la revendication 1 dans laquelle le système d'épreuve comporte une épreuve d'expression comprenant un acide nucléique de GFAT et l'agent de test candidat est un modulateur d'acide nucléique.

8. La méthode de la revendication 7 dans laquelle le modulateur d'acide nucléique est un oligomère antisens.

9. La méthode de la revendication 7 dans laquelle le modulateur d'acide nucléique est un PMO.

10. La méthode de la revendication 1 comprenant de plus :
(d) administrer l'agent de modulation de voie de l'Axine candidat identifié en (c) à un système modèle in vitro comprenant des cellules défectueuses en terme de fonction Axine et détecter un changement phénotypique dans le système modèle qui indique que la fonction Axine est restaurée.

11. Une méthode in vitro pour moduler une voie de l'Axine dans une cellule mammalienne comprenant mettre en contact la cellule avec un agent sélectionné dans le groupe consistant en un anticorps spécifique de GFAT, un oligomère antisens de GFAT ou une molécule ARNi de GFAT.

12. La méthode de la revendication 11, dans laquelle la cellule est défectueuse en terme de fonction Axine et grâce à laquelle la fonction Axine est restaurée lors du contact avec ledit agent.

13. Un anticorps spécifique de GFAT, un oligomère antisens de GFAT ou une molécule ARNi de GFAT destinés à être utilisés dans le traitement d'un cancer du côlon ou de la prostate.

14. L'anticorps spécifique de GFAT, l'oligomère antisens de GFAT ou la molécule ARNi de GFAT destinés à être utilisés dans le traitement d'un cancer du colon ou de la prostate de la revendication 13, lesquels doivent être administrés à un animal vertébré dans lequel un cancer a été décelé.

15. La méthode de la revendication 1, comprenant les étapes supplémentaires consistant à :
(d) fournir un deuxième système d'épreuve comprenant des cellules cultivées exprimant GFAT, dans laquelle la deuxième épreuve peut détecter un changement influencé par un agent de test dans la voie de l'Axine
(e) mettre en contact le deuxième système d'épreuve avec l'agent de test de (b) ou un agent dérivé de celui-ci dans des conditions dans auxquelles, n'était-ce la présence de l'agent de test ou de l'agent dérivé de celui-ci, le système fournit une activité de référence ; et
(f) détecter une activité influencée par l'agent du deuxième système d'épreuve, dans laquelle une différence entre l'activité influencée par l'agent et l'activité de référence du deuxième système d'épreuve confirme que l'agent de test ou l'agent dérivé de celui-ci est un agent de modulation de voie de l'Axine candidat.

16. La méthode de la revendication 15 dans laquelle le deuxième système d'épreuve est sélectionné dans le groupe consistant en un système d'épreuve d'apoptose, un système d'épreuve de prolifération cellulaire, un système d'épreuve d'angiogenèse, et un système d'épreuve d'induction hypoxique.

17. Une méthode in vitro pour diagnostiquer un cancer de la tête et du cou, du rein, du poumon, de la prostate, de la peau ou de l'utérus chez un patient comprenant :
(a) mettre un échantillon biologique prélevé chez le patient en contact avec une sonde pour l'expression de GFAT ;
(b) déterminer l'expression de GFAT d'après l'étape (a) et comparer les résultats avec un témoin ; et
(c) déterminer si la différence entre l'expression de GFAT dans l'échantillon du patient et le témoin tel que déterminée dans l'étape (b) indique un risque de cancer de la tête et du cou, du rein, du poumon, de la prostate, de la peau ou de l'utérus.

18. La méthode selon la revendication 17, dans laquelle ledit cancer est un cancer de la tête et du cou, du rein, ou de la peau.


### SEQUENCE LISTING

<110> EXELIXIS, INC.
<120> GFATS AS MODIFIERS OF THE AXIN PATHWAY AND METHODS OF USE
<130> EX06-020C-PC
<140> PCT/US2006/023971
   <141> 2006-06-20
<150> US 60/692,316
   <151> 2005-06-20
<160> 8
<170> PatentIn version 3.3
<210> 1
   <211> 3082
   <212> DNA
   <213> Homo sapiens
<220>
   <221> misc_feature
   <222> (1)..(3082)
   <223> M90516.1
<400> 1
   agggagtcgt gtcggcgcca ccccggcccc cgagcccgca gattgcccac cgaagctcgt     60
   gtgtgcaccc ccgatcccgc cagccactcg cccctggcct cgcgggccgt gtctccggca    120
   tcatgtgtgg tatatttgct tacttaaact accatgttcc tcgaacgaga cgagaaatcc    180
   tggagaccct aatcaaaggc cttcagagac tggagtacag aggatatgat tctgctggtg    240
   tgggatttga tggaggcaat gataaagatt gggaagccaa tgcctgcaaa acccagctta    300
   ttaagaagaa aggaaaagtt aaggcactgg atgaagaagt tcacaagcaa caagatatgg    360
   atttggatat agaatttgat gtacaccttg gaatagctca tacccgttgg gcaacacatg    420
   gagaacccag tcctgtcaat agccaccccc agcgctctga taaaaataat gaatttatcg    480
   ttattcacaa tggaatcatc accaactaca aagacttgaa aaagtttttg gaaagcaaag    540
   gctatgactt cgaatctgaa acagacacag agacaattgc caagctcgtt aagtatatgt    600
   atgacaatcg ggaaagtcaa gataccagct ttactacctt ggtggagaga gttatccaac    660
   aattggaagg tgcttttgca cttgtgttta aaagtgttca ttttcccggg caagcagttg    720
   gcacaaggcg aggtagccct ctgttgattg gtgtacggag tgaacataaa ctttctactg    780
   atcacattcc tatactctac agaacaggca aagacaagaa aggaagctgc aatctctctc    840
   gtgtggacag cacaacctgc cttttcccgg tggaagaaaa agcagtggag tattactttg    900
   cttctgatgc aagtgctgtc atagaacaca ccaatcgcgt catctttctg gaagatgatg    960
   atgttgcagc agtagtggat ggacgtcttt ctatccatcg aattaaacga actgcaggag   1020
   atcaccccgg acgagctgtg caaacactcc agatggaact ccagcagatc atgaagggca   1080
   acttcagttc atttatgcag aaggaaatat ttgagcagcc agagtctgtc gtgaacacaa   1140
   tgagaggaag agtcaacttt gatgactata ctgtgaattt gggtggtttg aaggatcaca 1200
   taaaggagat ccagagatgc cggcgtttga ttcttattgc ttgtggaaca agttaccatg 1260
   ctggtgtagc aacacgtcaa gttcttgagg agctgactga gttgcctgtg atggtggaac 1320
   tagcaagtga cttcctggac agaaacacac cagtctttcg agatgatgtt tgctttttcc 1380
   ttagtcaatc aggtgagaca gcagatactt tgatgggtct tcgttactgt aaggagagag 1440
   gagctttaac tgtggggatc acaaacacag ttggcagttc catatcacgg gagacagatt 1500
   gtggagttca tattaatgct ggtcctgaga ttggtgtggc cagtacaaag gcttatacca 1560
   gccagtttgt atcccttgtg atgtttgccc ttatgatgtg tgatgatcgg atctccatgc 1620
   aagaaagacg caaagagatc atgcttggat tgaaacggct gcctgatttg attaaggaag 1680
   tactgagcat ggatgacgaa attcagaaac tagcaacaga actttatcat cagaagtcag 1740
   ttctgataat gggacgaggc tatcattatg ctacttgtct tgaaggggca ctgaaaatca 1800
   aagaaattac ttatatgcac tctgaaggca tccttgctgg tgaattgaaa catggccctc 1860
   tggctttggt ggataaattg atgcctgtga tcatgatcat catgagagat cacacttatg 1920
   ccaagtgtca gaatgctctt cagcaagtgg ttgctcggca ggggcggcct gtggtaattt 1980
   gtgataagga ggatactgag accattaaga acacaaaaag aacgatcaag gtgccccact 2040
   cagtggactg cttgcagggc attctcagcg tgatcccttt acagttgctg gctttccacc 2100
   ttgctgtgct gagaggctat gatgttgatt tcccacggaa tcttgccaaa tctgtgactg 2160
   tagagtgagg aatatctata caaaatgtac gaaactgtat gattaagcaa cacaagacac 2220
   cttttgtatt taaaaccttg atttaaaata tcaccccttg aagccttttt ttagtaaatc 2280
   cttatttata tatcagttat aattattcca ctcaatatgt gatttttgtg aagttacctc 2340
   ttacattttc ccagtaattt gtggaggact ttgaataatg gaatctatat tggaatctgt 2400
   atcagaaaga ttctagctat tattttcttt aaagaatgct gggtgttgca tttctggacc 2460
   ctccacttca atctgagaag acaatatgtt tctaaaaatt ggtacttgtt tcaccatact 2520
   tcattcagac cagtgaaaga gtagtgcatt taattggagt atctaaagcc agtggcagtg 2580
   tatgctcata cttggacagt tagggaaggg tttgccaagt tttaagagaa gatgtgattt 2640
   attttgaaat ttgtttctgt tttgttttta aatcaaactg taaaacttaa aactgaaaaa 2700
   ttttattggt aggatttata tctaagtttg gttagcctta gtttctcaga cttgttgtct 2760
   attatctgta ggtggaagaa atttaggaag cgaaatatta cagtagtgca ttggtgggtc 2820
   tcaatcctta acatatttgc acaattttat agcacaaact ttaaattcaa gctgctttgg 2880
   acaactgaca atatgatttt aaatttgaag atgggatgtg tacatgttgg gtatcctact 2940
   actttgtgtt ttcatctcct aaaagtgttt tttatttcct tgtatctgta gtcttttatt 3000
   ttttaaatga ctgctgaatg acatatttta tcttgttctt taaaatcaca acacagagct 3060
   gctattaaat taatattgat at 3082
<210> 2
   <211> 3082
   <212> DNA
   <213> Homo sapiens
<220>
   <221> misc_feature
   <222> (1)..(3082)
   <223> NM_002056.1
<400> 2
   agggagtcgt gtcggcgcca ccccggcccc cgagcccgca gattgcccac cgaagctcgt      60
   gtgtgcaccc ccgatcccgc cagccactcg cccctggcct cgcgggccgt gtctccggca     120
   tcatgtgtgg tatatttgct tacttaaact accatgttcc tcgaacgaga cgagaaatcc     180
   tggagaccct aatcaaaggc cttcagagac tggagtacag aggatatgat tctgctggtg     240
   tgggatttga tggaggcaat gataaagatt gggaagccaa tgcctgcaaa acccagctta     300
   ttaagaagaa aggaaaagtt aaggcactgg atgaagaagt tcacaagcaa caagatatgg     360
   atttggatat agaatttgat gtacaccttg gaatagctca tacccgttgg gcaacacatg     420
   gagaacccag tcctgtcaat agccaccccc agcgctctga taaaaataat gaatttatcg     480
   ttattcacaa tggaatcatc accaactaca aagacttgaa aaagtttttg gaaagcaaag     540
   gctatgactt cgaatctgaa acagacacag agacaattgc caagctcgtt aagtatatgt     600
   atgacaatcg ggaaagtcaa gataccagct ttactacctt ggtggagaga gttatccaac     660
   aattggaagg tgcttttgca cttgtgttta aaagtgttca ttttcccggg caagcagttg     720
   gcacaaggcg aggtagccct ctgttgattg gtgtacggag tgaacataaa ctttctactg     780
   atcacattcc tatactctac agaacaggca aagacaagaa aggaagctgc aatctctctc     840
   gtgtggacag cacaacctgc cttttcccgg tggaagaaaa agcagtggag tattactttg     900
   cttctgatgc aagtgctgtc atagaacaca ccaatcgcgt catctttctg gaagatgatg     960
   atgttgcagc agtagtggat ggacgtcttt ctatccatcg aattaaacga actgcaggag    1020
   atcaccccgg acgagctgtg caaacactcc agatggaact ccagcagatc atgaagggca    1080
   acttcagttc atttatgcag aaggaaatat ttgagcagcc agagtctgtc gtgaacacaa    1140
   tgagaggaag agtcaacttt gatgactata ctgtgaattt gggtggtttg aaggatcaca    1200
   taaaggagat ccagagatgc cggcgtttga ttcttattgc ttgtggaaca agttaccatg    1260
   ctggtgtagc aacacgtcaa gttcttgagg agctgactga gttgcctgtg atggtggaac    1320
   tagcaagtga cttcctggac agaaacacac cagtctttcg agatgatgtt tgctttttcc   1380
   ttagtcaatc aggtgagaca gcagatactt tgatgggtct tcgttactgt aaggagagag    1440
   gagctttaac tgtggggatc acaaacacag ttggcagttc catatcacgg gagacagatt    1500
   gtggagttca tattaatgct ggtcctgaga ttggtgtggc cagtacaaag gcttatacca    1560
   gccagtttgt atcccttgtg atgtttgccc ttatgatgtg tgatgatcgg atctccatgc    1620
   aagaaagacg caaagagatc atgcttggat tgaaacggct gcctgatttg attaaggaag    1680
   tactgagcat ggatgacgaa attcagaaac tagcaacaga actttatcat cagaagtcag    1740
   ttctgataat gggacgaggc tatcattatg ctacttgtct tgaaggggca ctgaaaatca 1800
   aagaaattac ttatatgcac tctgaaggca tccttgctgg tgaattgaaa catggccctc 1860
   tggctttggt ggataaattg atgcctgtga tcatgatcat catgagagat cacacttatg 1920
   ccaagtgtca gaatgctctt cagcaagtgg ttgctcggca ggggcggcct gtggtaattt 1980
   gtgataagga ggatactgag accattaaga acacaaaaag aacgatcaag gtgccccact 2040
   cagtggactg cttgcagggc attctcagcg tgatcccttt acagttgctg gctttccacc 2100
   ttgctgtgct gagaggctat gatgttgatt tcccacggaa tcttgccaaa tctgtgactg 2160
   tagagtgagg aatatctata caaaatgtac gaaactgtat gattaagcaa cacaagacac 2220
   cttttgtatt taaaaccttg atttaaaata tcaccccttg aagccttttt ttagtaaatc 2280
   cttatttata tatcagttat aattattcca ctcaatatgt gatttttgtg aagttacctc 2340
   ttacattttc ccagtaattt gtggaggact ttgaataatg gaatctatat tggaatctgt 2400
   atcagaaaga ttctagctat tattttcttt aaagaatgct gggtgttgca tttctggacc 2460
   ctccacttca atctgagaag acaatatgtt tctaaaaatt ggtacttgtt tcaccatact 2520
   tcattcagac cagtgaaaga gtagtgcatt taattggagt atctaaagcc agtggcagtg 2580
   tatgctcata cttggacagt tagggaaggg tttgccaagt tttaagagaa gatgtgattt 2640
   attttgaaat ttgtttctgt tttgttttta aatcaaactg taaaacttaa aactgaaaaa 2700
   ttttattggt aggatttata tctaagtttg gttagcctta gtttctcaga cttgttgtct 2760
   attatctgta ggtggaagaa atttaggaag cgaaatatta cagtagtgca ttggtgggtc 2820
   tcaatcctta acatatttgc acaattttat agcacaaact ttaaattcaa gctgctttgg 2880
   acaactgaca atatgatttt aaatttgaag atgggatgtg tacatgttgg gtatcctact 2940
   actttgtgtt ttcatctcct aaaagtgttt tttatttcct tgtatctgta gtcttttatt 3000
   ttttaaatga ctgctgaatg acatatttta tcttgttctt taaaatcaca acacagagct 3060
   gctattaaat taatattgat at 3082
<210> 3
   <211> 3014
   <212> DNA
   <213> Homo sapiens
<220>
   <221> misc_feature
   <222> (1)..(3014)
   <223> NM_005110.1
<400> 3
   gcggagccca cggagcccac ggaggagccc acggaggagc cccagcgtcc gaacgggcag 60
   accccctcga gccgcgaagg agcccgagaa gcagccacga tgtgcggaat ctttgcctac 120
   atgaactaca gagtcccccg gacgaggaag gagatcttcg aaaccctcat caagggcctg 180
   cagcggctgg agtacagagg ctacgactcg gcaggtgtgg cgatcgatgg gaataatcac 240
   gaagtcaaag aaagacacat tcagctggtc aagaaaaggg ggaaagtcaa ggctctcgat 300
   gaagaacttt acaaacaaga cagcatggac ttaaaagtgg agtttgagac acacttcggc 360
   attgcccaca cgcgctgggc cacccacggg gtccccagtg ctgtcaacag ccaccctcag 420
   cgctcagaca aaggcaacga atttgttgtc atccacaatg ggatcatcac aaattacaaa 480
   gatctgagga aatttctgga aagcaaaggc tacgagtttg agtcagaaac agatacagag 540
   accatcgcca agctgattaa atatgtgttc gacaacagag aaactgagga cattacgttt 600
   tcaacgttgg tcgagagagt cattcagcag ttggaaggtg cattcgcgct ggttttcaag 660
   agtgtccact acccaggaga agccgttgcc acacggagag gcagccccct gctcatcgga 720
   gtccggagca aatacaagct ctccacagaa cagatcccta tcttatacag gacgtgcact 780
   ctggagaatg tgaagaatat ctgtaagaca cggatgaaga ggctggacag ctccgcctgc 840
   ctgcatgctg tgggcgacaa ggccgtggaa ttcttctttg cttctgatgc aagcgctatc 900
   atagagcaca ccaaccgggt catcttcctg gaggacgatg acatcgccgc agtggctgat 960
   gggaaactct ccattcaccg ggtcaagcgc tcggccagtg atgacccatc tcgagccatc 1020
   cagaccttgc agatggaact gcagcaaatc atgaaaggta acttcagtgc gtttatgcag 1080
   aaggagatct tcgaacagcc agaatcagtt ttcaatacta tgagaggtcg ggtgaatttt 1140
   gaaaccaaca cagtgctcct gggtggcttg aaggaccact tgaaggagat tcgacgatgc 1200
   cgacggctca tcgtgattgg ctgtggaacc agctaccacg ctgccgtggc tacgcggcaa 1260
   gttttggagg aactgactga gcttcctgtg atggttgaac ttgctagtga ttttctggac 1320
   aggaacacac ctgtgttcag ggatgacgtt tgctttttca tcagccagtc aggcgagacc 1380
   gcggacaccc tcctggcgct gcgctactgt aaggaccgcg gcgctctcac cgtgggcgtc 1440
   accaacaccg tgggcagctc catctctcgc gagaccgact gcggcgtcca catcaacgca 1500
   gggccggaga tcggcgtggc cagcaccaag gcttatacca gtcagttcat ctctctggtg 1560
   atgtttggtt tgatgatgtc tgaagaccga atttcactac aaaacaggag gcaagagatc 1620
   atccgtggct tgagatcttt acctgagctg atcaaggaag tgctgtctct ggaggagaag 1680
   atccacgact tggccctgga gctctacacg cagagatcgc tgctggtgat ggggcggggc 1740
   tacaactatg ccacctgcct ggaaggagcc ctgaaaatta aagagataac ctacatgcac 1800
   tcagaaggca tcctggctgg ggagctgaag cacgggcccc tggcactgat tgacaagcag 1860
   atgcccgtca tcatggtcat tatgaaggat ccttgcttcg ccaaatgcca gaacgccctg 1920
   cagcaagtca cggcccgcca gggtcgcccc attatactgt gctccaagga cgatactgaa 1980
   agttccaagt ttgcgtataa gacaattgag ctgccccaca ctgtggactg cctccagggc 2040
   atcctgagcg tgattccgct gcagctgctg tccttccacc tggctgttct ccgaggatat 2100
   gacgttgact tccccagaaa tctggccaag tctgtaactg tggaatgagg ctgagaccgt 2160
   gacaagacca tcaccacctt tcatctgatt ccagacctgt cccaacagca gggatgctac 2220
   atgggaagag aagtggacat cccacatgtt ctgcgtgctc ctgtagagct tgacagcttc 2280
   cacgtgcctt ctacccaagt gcttttgctt acagcagata ctgtttctct gtgtcctgaa    2340
   gtcgccagag gagaagggaa tcattgttta cacatgggga tcagagcaga cttctccact    2400
   actgtgcaat agagatacag ctctcttcag agtaactgtg aaccttttat aaccaacact     2460
   agagttagtt ttaaaagaca agatatttat aatgacgact gtatagcttt taagttattt    2520
   ttctagtatg tggctttctg tagccgtggt aacggccaaa ctgttcatcc tagctaccca    2580
   tgctctgtgt ccaggcttgc tcctggcagg tggcattcat ctcagatgtg agcacaaggc    2640
   attggccctc tggactcctt tctccttttc tttcctctct aggctgctcc tgaatcctgt    2700
   tctctgacat ccgtggagcc cctcctgcat ccacctatgc ctcctataag tccagttgaa    2760
   atctcagcct ccttcaacat tttcttctcg tgtgtggccc acatccctcc acttctccaa    2820
   cttctgttta atctgatcac ggctcttttt aagccctggc agcattttgg tccctgctcc    2880
   ttgcccatag taaaacagct tgaaatatcc catgcaagag agtagtttca agtgggcgac    2940
   tctgctctct atttaaaagc gtgcacaatc aaaagtacta tgcaatttta ggacaataaa    3000
   gaacatacag ttcc                                                      3014
<210> 4
   <211> 3024
   <212> DNA
   <213> Homo sapiens
<220>
   <221> misc_feature
   <222> (1)..(3024)
   <223> AK022507.1
<400> 4
   agctggagcc cgcggagccc acggagccca cggaggagcc cacggaggag ccccagcgtc 60
   cgaacgggca gaccccctcg agccgcgaag gagcccgaga agcagccacg atgtgcggaa 120
   tctttgccta catgaaccac agagtccccc ggacgaggaa ggagatcttc gaaaccctca 180
   tcaagggcct gcagcggctg gagtacagag gctacgactc ggcaggtgtg gcgatcgatg 240
   ggaataatca cgaagtcaaa gaaagacaca ttcagctggt caagaaaagg gggaaagtca 300
   aggctctcga tgaagaactt tacaaacaag acagcatgga cttaaaagtg gagtttgaga 360
   cacacttcgg cattgcccac acgcgctggg ccacccacgg ggtccccagt gctgtcaaca 420
   gccaccctca gcgctcagac aaaggcaacg aatttgttgt catccacaat gggatcatca 480
   caaattacaa agatctgagg aaatttctgg aaagcaaagg ctacgagttt gagtcagaaa 540
   cagatacaga gaccatcgcc aagctgatta aatatgtgtt cgacaacaga gaaactgagg 600
   acattacgtt ttcaacgttg gtcgagagag tcattcagca gttggaaggt gcattcgcgc 660
   tggttttcaa gagtgtccac tacccaggag aagccgttgc cacacggaga ggcagccccc 720
   tgctcatcgg agtccggagc aaatacaagc tctccacaga acagatccct atcttataca 780
   ggacgtgcac tctggagaat gtggagaata tctgtaagac acggatgaag aggctggaca 840
   gctccgcctg cctgcatgct gtgggcgaca aggccgtgga attcttcttt gcttctgatg 900
   caagcgctat catagagcac accaaccggg tcatcttcct ggaggacgat gacatcgccg    960
   cagtggctga tgggaaactc tccattcacc gggtcaagcg ctcggccagt gatgacccat   1020
   ctcgagccat ccagaccttg cagatggaac tgcagcaaat catgaaaggt aacttcagtg   1080
   cgtttatgca gaaggagatc ttcgaacagc cagaatccgt tttcaatact atgagaggtc   1140
   gggtgaattt tgaaaccaac acagtgctcc tgggtggctt gaaggaccac ttgaaggaga   1200
   ttcgacgatg ccgacggctc atcgtgattg gctgtggaac cagctaccac gctgccgtgg   1260
   ctacgcggca agttttggag gaactgactg agcttcctgt gatggttgaa cttgctagtg   1320
   attttctgga caggaacaca cctgtgttca gggatgacgt ttgctttttc atcagccagt   1380
   caggcgagac cgcggacacc ctcctggcgc tgcgctactg taaggaccgc ggcgctctca   1440
   ccgtgggcgt caccaacacc gtgggcagct ccatctctcg cgagaccgac tgcggcgtcc   1500
   acatcaacgc agggccggag atcggcgtgg ccagcaccaa ggcttatacc agtcagttca   1560
   tctctctggt gatgtttggt ttgatgatgt ctgaagaccg aatttcacta caaaacagga   1620
   ggcaagagat catccgtggc ttgagatctt tacctgagct gatcaaggaa gtgctgtctc   1680
   tggaggagaa gatccacgac ttggccctgg agctctacac gcagagatcg ctgctggtga   1740
   tggggcgggg ctacaactat gccacctgcc tggaaggagc cctgaaaatt aaagagataa   1800
   cctacatgca ctcagaaggc atcctggctg gggagctgaa gcacgggccc ctggcactga   1860
   ttgacaagca gatgcccgtc atcatggtca ttatgaagga tccttgcttc gccaaatgcc   1920
   agaacgccct gcagcaagtc acggcccgcc agggtcgccc cattatactg tgctccaagg   1980
   acgatactga aagttccaag tttgcgtata agacaatcga gctgccccac actgtggact   2040
   gcctccaggg catcctgagc gtgattccgc tgcagctgct gtccttccac ctggctgttc   2100
   tccgaggata tgacgttgac ttccccagaa atctggccaa gtctgtaact gtggaatgag   2160
   gctgagaccg tgacaagacc atcaccacct ttcatctgat tccagacctg tcccaacagc   2220
   agggatgcta catgggaaga gaagtggaca tcccacatgt tctgcgtgct cctgtagagc   2280
   ttgacagctt ccacgtgcct tctacccaag tgcttttgct tacagcagat actgtttctc   2340
   tgtgtcctga agtcgccaga ggagaaggga atcattgttt acacatgggg atcagagcag   2400
   acttctccac tactgtgcaa tagagataca gctctcttca gagtaactgt gaacctttta   2460
   taaccaacac tagagttagt tttaaaagac aagatattta taatgacgac tgtatagctt   2520
   ttaagttatt tttctagtat gtggctttct gtagccgtgg taacggccaa actgttcatc   2580
   ctagctaccc atgctctgtg tccaggcttg ctcctggcag gtggcattca tctcagatgt   2640
   gagcacaagg cattggccct ctggactcct ttctcctttt ctttcctctc taggctgctc   2700
   ctgaatcctg ttctctgaca tccgtggagc ccctcctgca tccacctatg cctcctatag   2760
   gtccagttga aatctcagcc tccttcaaca ttttcttctc gtgtgtggcc cacatccctc   2820
   cacttctcca acttctgttt aatctgatca cggctctttt taagccctgg cagcattttg   2880
   gtccctgctc cttgcccata gtaaaacagc ttgaaatatc ccatgcaaga gagtagtttc   2940
   aggtgggcaa ctctgctctc tatttaaaag cgtgcacaat caaaagtact atgcaatttt 3000
   aggacaataa agaacataca gttt 3024
<210> 5
   <211> 3062
   <212> DNA
   <213> Homo sapiens
<220>
   <221> misc_feature
   <222> (1)..(3062)
   <223> BC000012.1
<400> 5
   ggcacgaggg cggagcccac ggagcccacg gagcccacgg aggagcccac ggaggagccc      60
   cagcgtccga acgggcagac cccctcgagc cgcgaaggag cccgagaagc agccacgatg     120
   tgcggaatct ttgcctacat gaactacaga gtcccccgga cgaggaagga gatcttcgaa     180
   accctcatca agggcctgca gcggctggag tacagaggct acgactcggc aggtgtggcg     240
   atcgatggga ataatcacga agtcaaagaa agacacattc agctggtcaa gaaaaggggg     300
   aaagtcaagg ctctcgatga agaactttac aaacaagaca gcatggactt aaaagtggag     360
   tttgagacac acttcggcat tgcccacacg cgctgggcca cccacggggt ccccagtgct     420
   gtcaacagcc accctcagcg ctcagacaaa ggcaacgaat ttgttgtcat ccacaatggg     480
   atcatcacaa attacaaaga tctgaggaaa tttctggaaa gcaaaggcta cgagtttgag     540
   tcagaaacag atacagagac catcgccaag ctgattaaat atgtgttcga caacagagaa     600
   actgaggaca ttacgttttc aacgttggtc gagagagtca ttcagcagtt ggaaggtgca     660
   ttcgcgctgg ttttcaagag tgtccactac ccaggagaag ccgttgccac acggagaggc     720
   agccccctgc tcatcggagt ccggagcaaa tacaagctct ccacagaaca gatccctatc     780
   ttatacagga cgtgcactct ggagaatgtg aagaatatct gtaagacacg gatgaagagg     840
   ctggacagct ccgcctgcct gcatgctgtg ggcgacaagg ccgtggaatt cttctttgct     900
   tctgatgcaa gcgctatcat agagcacacc aaccgggtca tcttcctgga ggacgatgac     960
   atcgccgcag tggctgatgg gaaactctcc attcaccggg tcaagcgctc ggccagtgat    1020
   gacccatctc gagccatcca gaccttgcag atggaactgc agcaaatcat gaaaggtaac    1080
   ttcagtgcgt ttatgcagaa ggagatcttc gaacagccag aatcagtttt caatactatg    1140
   agaggtcggg tgaattttga aaccaacaca gtgctcctgg gtggcttgaa ggaccacttg    1200
   aaggagattc gacgatgccg acggctcatc gtgattggct gtggaaccag ctaccacgct    1260
   gccgtggcta cgcggcaagt tttggaggaa ctgactgagc ttcctgtgat ggttgaactt    1320
   gctagtgatt ttctggacag gaacacacct gtgttcaggg atgacgtttg ctttttcatc    1380
   agccagtcag gcgagaccgc ggacaccctc ctggcgctgc gctactgtaa ggaccgcggc    1440
   gctctcaccg tgggcgtcac caacaccgtg ggcagctcca tctctcgcga gaccgactgc    1500
   ggcgtccaca tcaacgcagg gccggaggtc ggcgtggcca gcaccaaggc ttataccagt   1560
   cagttcatct ctctggtgat gtttggtttg atgatgtctg aagaccgaat ttcactacaa   1620
   aacaggaggc aagagatcat ccgtggcttg agatctttac ctgagctgat caaggaagtg   1680
   ctgtctctgg aggagaagat ccacgacttg gccctggagc tctacacgca gagatcgctg   1740
   ctggtgatgg ggcggggcta caactatgcc acctgcctgg aaggagccct gaaaattaaa   1800
   gagataacct acatgcactc agaaggcatc ctggctgggg agctgaagca cgggcccctg   1860
   gcactgattg acaagcagat gcccgtcatc atggtcatta tgaaggatcc ttgcttcgcc   1920
   aaatgccaga acgccctgca gcaagtcacg gcccgccagg gtcgccccat tatactgtgc   1980
   tccaaggacg atactgaaag ttccaagttt gcgtataaga caatcgagct gccccacact   2040
   gtggactgcc tccagggcat cctgagcgtg attccgctgc agctgctgtc cttccacctg   2100
   gctgttctcc gaggatatga cgttgacttc cccagaaatc tggccaagtc tgtaactgtg   2160
   gaatgaggct gagaccgtga caagaccatc accacctttc atctgattcc agacctgtcc   2220
   caacagcagg gatgctacat gggaagagaa gtggacatcc cacatgttct gcgtgctcct   2280
   gtagagcttg acagcttcca cgtgccttct acccaagtgc ttttgcttac agcagatact   2340
   gtttctctgt gtcctgaagt cgccagagga gaagggaatc attgtttaca catggggatc   2400
   agagcagact tctccactac tgtgcaatag agatacagct ctcttcagag taactgtgaa   2460
   ccttttataa ccaacactag agttagtttt aaaagacaag atatttataa tgacgactgt   2520
   atagctttta agttattttt ctagtatgtg gctttctgta gccgtggtaa cggccaaact   2580
   gttcatccta gctacccatg ctctgtgtcc aggcttgctc ctggcaggtg gcattcatct   2640
   cagatgtgag cacaaggcat tggccctctg gactcctttc tccttttctt tcctctctag   2700
   gctgctcctg aatcctgttc tctgacatcc gtggagcccc tcctgcatcc acctatgcct   2760
   cctataagtc caattgaaat ctcagcctcc ttcaacattt tcttctcgtg tgtggcccac   2820
   atccctccac ttctccaact tctgtttaat ctgatcacgg ctctttttaa gccctggcag   2880
   cattttggtc cctgctcctt gcccatagta aaacagcttg aaatatccca tgcaagagag   2940
   tagtttcaag tgggcaactc tgctctctat ttaaaagcgt gcacaatcaa aagtactatg   3000
   caattttagg acaataaaga acatacagtt tttttgtgtg aaaaaaaaaa aaaaaaaaaa   3060
   aa                                                                  3062
<210> 6
   <211> 681
   <212> PRT
   <213> Homo sapiens
<220>
   <221> MISC_FEATURE
   <222> (1)..(681)
   <223> Q06210
<400> 6
   Met Cys Gly Ile Phe Ala Tyr Leu Asn Tyr His Val Pro Arg Thr Arg
   1               5                   10                  15
   Arg Glu Ile Leu Glu Thr Leu Ile Lys Gly Leu Gln Arg Leu Glu Tyr
               20                  25                  30
   Arg Gly Tyr Asp Ser Ala Gly Val Gly Phe Asp Gly Gly Asn Asp Lys
           35                  40                  45
   Asp Trp Glu Ala Asn Ala Cys Lys Thr Gln Leu Ile Lys Lys Lys Gly
       50                  55                  60
   Lys Val Lys Ala Leu Asp Glu Glu Val His Lys Gln Gln Asp Met Asp
   65                  70                  75                  80
   Leu Asp Ile Glu Phe Asp Val His Leu Gly Ile Ala His Thr Arg Trp
                   85                  90                  95
   Ala Thr His Gly Glu Pro Ser Pro Val Asn Ser His Pro Gln Arg Ser
               100                 105                 110
   Asp Lys Asn Asn Glu Phe Ile Val Ile His Asn Gly Ile Ile Thr Asn
           115                 120                 125
   Tyr Lys Asp Leu Lys Lys Phe Leu Glu Ser Lys Gly Tyr Asp Phe Glu
       130                 135                 140
   Ser Glu Thr Asp Thr Glu Thr Ile Ala Lys Leu Val Lys Tyr Met Tyr
   145                 150                 155                 160
   Asp Asn Arg Glu Ser Gln Asp Thr Ser Phe Thr Thr Leu Val Glu Arg
                   165                 170                 175
   Val Ile Gln Gln Leu Glu Gly Ala Phe Ala Leu Val Phe Lys Ser Val
               180                 185                 190
   His Phe Pro Gly Gln Ala Val Gly Thr Arg Arg Gly Ser Pro Leu Leu
           195                 200                 205
   Ile Gly Val Arg Ser Glu His Lys Leu Ser Thr Asp His Ile Pro Ile
       210                 215                 220
   Leu Tyr Arg Thr Gly Lys Asp Lys Lys Gly Ser Cys Asn Leu Ser Arg
   225                 230                 235                 240
   Val Asp Ser Thr Thr Cys Leu Phe Pro Val Glu Glu Lys Ala Val Glu
                   245                 250                 255
   Tyr Tyr Phe Ala Ser Asp Ala Ser Ala Val Ile Glu His Thr Asn Arg
               260                 265                 270
   Val Ile Phe Leu Glu Asp Asp Asp Val Ala Ala Val Val Asp Gly Arg
   275 280 285
   Leu Ser Ile His Arg Ile Lys Arg Thr Ala Gly Asp His Pro Gly Arg
   290 295 300
   Ala Val Gln Thr Leu Gln Met Glu Leu Gln Gln Ile Met Lys Gly Asn
   305 310 315 320
   Phe Ser Ser Phe Met Gln Lys Glu Ile Phe Glu Gln Pro Glu Ser Val
   325 330 335
   Val Asn Thr Met Arg Gly Arg Val Asn Phe Asp Asp Tyr Thr Val Asn
   340 345 350
   Leu Gly Gly Leu Lys Asp His Ile Lys Glu Ile Gln Arg Cys Arg Arg
   355 360 365
   Leu Ile Leu Ile Ala Cys Gly Thr Ser Tyr His Ala Gly Val Ala Thr
   370 375 380
   Arg Gln Val Leu Glu Glu Leu Thr Glu Leu Pro Val Met Val Glu Leu
   385 390 395 400
   Ala Ser Asp Phe Leu Asp Arg Asn Thr Pro Val Phe Arg Asp Asp Val
   405 410 415
   Cys Phe Phe Leu Ser Gln Ser Gly Glu Thr Ala Asp Thr Leu Met Gly
   420 425 430
   Leu Arg Tyr Cys Lys Glu Arg Gly Ala Leu Thr Val Gly Ile Thr Asn
   435 440 445
   Thr Val Gly Ser Ser Ile Ser Arg Glu Thr Asp Cys Gly Val His Ile
   450 455 460
   Asn Ala Gly Pro Glu Ile Gly Val Ala Ser Thr Lys Ala Tyr Thr Ser
   465 470 475 480
   Gln Phe Val Ser Leu Val Met Phe Ala Leu Met Met Cys Asp Asp Arg
   485 490 495
   Ile Ser Met Gln Glu Arg Arg Lys Glu Ile Met Leu Gly Leu Lys Arg
   500 505 510
   Leu Pro Asp Leu Ile Lys Glu Val Leu Ser Met Asp Asp Glu Ile Gln
   515 520 525
   Lys Leu Ala Thr Glu Leu Tyr His Gln Lys Ser Val Leu Ile Met Gly
   530 535 540
   Arg Gly Tyr His Tyr Ala Thr Cys Leu Glu Gly Ala Leu Lys Ile Lys
   545 550 555 560
   Glu Ile Thr Tyr Met His Ser Glu Gly Ile Leu Ala Gly Glu Leu Lys
   565 570 575
   His Gly Pro Leu Ala Leu Val Asp Lys Leu Met Pro Val Ile Met Ile
   580 585 590
   Ile Met Arg Asp His Thr Tyr Ala Lys Cys Gln Asn Ala Leu Gln Gln
   595 600 605
   Val Val Ala Arg Gln Gly Arg Pro Val Val Ile Cys Asp Lys Glu Asp
   610 615 620
   Thr Glu Thr Ile Lys Asn Thr Lys Arg Thr Ile Lys Val Pro His Ser
   625 630 635 640
   Val Asp Cys Leu Gln Gly Ile Leu Ser Val Ile Pro Leu Gln Leu Leu
   645 650 655
   Ala Phe His Leu Ala Val Leu Arg Gly Tyr Asp Val Asp Phe Pro Arg
   660 665 670
   Asn Leu Ala Lys Ser Val Thr Val Glu
   675 680
<210> 7
   <211> 681
   <212> PRT
   <213> Homo sapiens
<220>
   <221> MISC_FEATURE
   <222> (1)..(681)
   <223> NP_002047.1
<400> 7
   Met Cys Gly Ile Phe Ala Tyr Leu Asn Tyr His Val Pro Arg Thr Arg
   1               5                   10                  15
   Arg Glu Ile Leu Glu Thr Leu Ile Lys Gly Leu Gln Arg Leu Glu Tyr
               20                  25                  30
   Arg Gly Tyr Asp Ser Ala Gly Val Gly Phe Asp Gly Gly Asn Asp Lys
           35                  40                  45
   Asp Trp Glu Ala Asn Ala Cys Lys Thr Gln Leu Ile Lys Lys Lys Gly
       50                  55                  60
   Lys Val Lys Ala Leu Asp Glu Glu Val His Lys Gln Gln Asp Met Asp
   65                  70                  75                  80
   Leu Asp Ile Glu Phe Asp Val His Leu Gly Ile Ala His Thr Arg Trp
   85 90 95
   Ala Thr His Gly Glu Pro Ser Pro Val Asn Ser His Pro Gln Arg Ser
   100 105 110
   Asp Lys Asn Asn Glu Phe Ile Val Ile His Asn Gly Ile Ile Thr Asn
   115 120 125
   Tyr Lys Asp Leu Lys Lys Phe Leu Glu Ser Lys Gly Tyr Asp Phe Glu
   130 135 140
   Ser Glu Thr Asp Thr Glu Thr Ile Ala Lys Leu Val Lys Tyr Met Tyr
   145 150 155 160
   Asp Asn Arg Glu Ser Gln Asp Thr Ser Phe Thr Thr Leu Val Glu Arg
   165 170 175
   Val Ile Gln Gln Leu Glu Gly Ala Phe Ala Leu Val Phe Lys Ser Val
   180 185 190
   His Phe Pro Gly Gln Ala Val Gly Thr Arg Arg Gly Ser Pro Leu Leu
   195 200 205
   Ile Gly Val Arg Ser Glu His Lys Leu Ser Thr Asp His Ile Pro Ile
   210 215 220
   Leu Tyr Arg Thr Gly Lys Asp Lys Lys Gly Ser Cys Asn Leu Ser Arg
   225 230 235 240
   Val Asp Ser Thr Thr Cys Leu Phe Pro Val Glu Glu Lys Ala Val Glu
   245 250 255
   Tyr Tyr Phe Ala Ser Asp Ala Ser Ala Val Ile Glu His Thr Asn Arg
   260 265 270
   Val Ile Phe Leu Glu Asp Asp Asp Val Ala Ala Val Val Asp Gly Arg
   275 280 285
   Leu Ser Ile His Arg Ile Lys Arg Thr Ala Gly Asp His Pro Gly Arg
   290 295 300
   Ala Val Gln Thr Leu Gln Met Glu Leu Gln Gln Ile Met Lys Gly Asn
   305 310 315 320
   Phe Ser Ser Phe Met Gln Lys Glu Ile Phe Glu Gln Pro Glu Ser Val
   325 330 335
   Val Asn Thr Met Arg Gly Arg Val Asn Phe Asp Asp Tyr Thr Val Asn
   340 345 350
   Leu Gly Gly Leu Lys Asp His Ile Lys Glu Ile Gln Arg Cys Arg Arg
           355                 360                 365
   Leu Ile Leu Ile Ala Cys Gly Thr Ser Tyr His Ala Gly Val Ala Thr
       370                 375                 380
   Arg Gln Val Leu Glu Glu Leu Thr Glu Leu Pro Val Met Val Glu Leu
   385                 390                 395                 400
   Ala Ser Asp Phe Leu Asp Arg Asn Thr Pro Val Phe Arg Asp Asp Val
                   405                 410                 415
   Cys Phe Phe Leu Ser Gln Ser Gly Glu Thr Ala Asp Thr Leu Met Gly
               420                 425                 430
   Leu Arg Tyr Cys Lys Glu Arg Gly Ala Leu Thr Val Gly Ile Thr Asn
           435                 440                 445
   Thr Val Gly Ser Ser Ile Ser Arg Glu Thr Asp Cys Gly Val His Ile
       450                 455                 460
   Asn Ala Gly Pro Glu Ile Gly Val Ala Ser Thr Lys Ala Tyr Thr Ser
   465                 470                 475                 480
   Gln Phe Val Ser Leu Val Met Phe Ala Leu Met Met Cys Asp Asp Arg
                   485                 490                 495
   Ile Ser Met Gln Glu Arg Arg Lys Glu Ile Met Leu Gly Leu Lys Arg
               500                 505                 510
   Leu Pro Asp Leu Ile Lys Glu Val Leu Ser Met Asp Asp Glu Ile Gln
           515                 520                 525
   Lys Leu Ala Thr Glu Leu Tyr His Gln Lys Ser Val Leu Ile Met Gly
       530                 535                 540
   Arg Gly Tyr His Tyr Ala Thr Cys Leu Glu Gly Ala Leu Lys Ile Lys
   545                 550                 555                 560
   Glu Ile Thr Tyr Met His Ser Glu Gly Ile Leu Ala Gly Glu Leu Lys
                   565                 570                 575
   His Gly Pro Leu Ala Leu Val Asp Lys Leu Met Pro Val Ile Met Ile
               580                 585                 590
   Ile Met Arg Asp His Thr Tyr Ala Lys Cys Gln Asn Ala Leu Gln Gln
           595                 600                 605
   Val Val Ala Arg Gln Gly Arg Pro Val Val Ile Cys Asp Lys Glu Asp
       610                 615                 620
   Thr Glu Thr Ile Lys Asn Thr Lys Arg Thr Ile Lys Val Pro His Ser
   625                 630                 635                 640
   Val Asp Cys Leu Gln Gly Ile Leu Ser Val Ile Pro Leu Gln Leu Leu
                   645                 650                 655
   Ala Phe His Leu Ala Val Leu Arg Gly Tyr Asp Val Asp Phe Pro Arg
               660                 665                 670
   Asn Leu Ala Lys Ser Val Thr Val Glu
           675                 680
<210> 8
   <211> 682
   <212> PRT
   <213> Homo sapiens
<220>
   <221> MISC_FEATURE
   <222> (1)..(682)
   <223> NP_005101.1
<400> 8
   Met Cys Gly Ile Phe Ala Tyr Met Asn Tyr Arg Val Pro Arg Thr Arg
   1               5                   10                  15
   Lys Glu Ile Phe Glu Thr Leu Ile Lys Gly Leu Gln Arg Leu Glu Tyr
               20                  25                  30
   Arg Gly Tyr Asp Ser Ala Gly Val Ala Ile Asp Gly Asn Asn His Glu
           35                  40                  45
   Val Lys Glu Arg His Ile Gln Leu Val Lys Lys Arg Gly Lys Val Lys
       50                  55                  60
   Ala Leu Asp Glu Glu Leu Tyr Lys Gln Asp Ser Met Asp Leu Lys Val
   65                  70                  75                  80
   Glu Phe Glu Thr His Phe Gly Ile Ala His Thr Arg Trp Ala Thr His
                   85                  90                  95
   Gly Val Pro Ser Ala Val Asn Ser His Pro Gln Arg Ser Asp Lys Gly
               100                 105                 110
   Asn Glu Phe Val Val Ile His Asn Gly Ile Ile Thr Asn Tyr Lys Asp
           115                 120                 125
   Leu Arg Lys Phe Leu Glu Ser Lys Gly Tyr Glu Phe Glu Ser Glu Thr
       130                 135                 140
   Asp Thr Glu Thr Ile Ala Lys Leu Ile Lys Tyr Val Phe Asp Asn Arg
   145                 150                 155                 160
   Glu Thr Glu Asp Ile Thr Phe Ser Thr Leu Val Glu Arg Val Ile Gln
   165 170 175
   Gln Leu Glu Gly Ala Phe Ala Leu Val Phe Lys Ser Val His Tyr Pro
   180 185 190
   Gly Glu Ala Val Ala Thr Arg Arg Gly Ser Pro Leu Leu Ile Gly Val
   195 200 205
   Arg Ser Lys Tyr Lys Leu Ser Thr Glu Gln Ile Pro Ile Leu Tyr Arg
   210 215 220
   Thr Cys Thr Leu Glu Asn Val Lys Asn Ile Cys Lys Thr Arg Met Lys
   225 230 235 240
   Arg Leu Asp Ser Ser Ala Cys Leu His Ala Val Gly Asp Lys Ala Val
   245 250 255
   Glu Phe Phe Phe Ala Ser Asp Ala Ser Ala Ile Ile Glu His Thr Asn
   260 265 270
   Arg Val Ile Phe Leu Glu Asp Asp Asp Ile Ala Ala Val Ala Asp Gly
   275 280 285
   Lys Leu Ser Ile His Arg Val Lys Arg Ser Ala Ser Asp Asp Pro Ser
   290 295 300
   Arg Ala Ile Gln Thr Leu Gln Met Glu Leu Gln Gln Ile Met Lys Gly
   305 310 315 320
   Asn Phe Ser Ala Phe Met Gln Lys Glu Ile Phe Glu Gln Pro Glu Ser
   325 330 335
   Val Phe Asn Thr Met Arg Gly Arg Val Asn Phe Glu Thr Asn Thr Val
   340 345 350
   Leu Leu Gly Gly Leu Lys Asp His Leu Lys Glu Ile Arg Arg Cys Arg
   355 360 365
   Arg Leu Ile Val Ile Gly Cys Gly Thr Ser Tyr His Ala Ala Val Ala
   370 375 380
   Thr Arg Gln Val Leu Glu Glu Leu Thr Glu Leu Pro Val Met Val Glu
   385 390 395 400
   Leu Ala Ser Asp Phe Leu Asp Arg Asn Thr Pro Val Phe Arg Asp Asp
   405 410 415
   Val Cys Phe Phe Ile Ser Gln Ser Gly Glu Thr Ala Asp Thr Leu Leu
   420 425 430
   Ala Leu Arg Tyr Cys Lys Asp Arg Gly Ala Leu Thr Val Gly Val Thr
   435 440 445
   Asn Thr Val Gly Ser Ser Ile Ser Arg Glu Thr Asp Cys Gly Val His
   450 455 460
   Ile Asn Ala Gly Pro Glu Ile Gly Val Ala Ser Thr Lys Ala Tyr Thr
   465 470 475 480
   Ser Gln Phe Ile Ser Leu Val Met Phe Gly Leu Met Met Ser Glu Asp
   485 490 495
   Arg Ile Ser Leu Gln Asn Arg Arg Gln Glu Ile Ile Arg Gly Leu Arg
   500 505 510
   Ser Leu Pro Glu Leu Ile Lys Glu Val Leu Ser Leu Glu Glu Lys Ile
   515 520 525
   His Asp Leu Ala Leu Glu Leu Tyr Thr Gln Arg Ser Leu Leu Val Met
   530 535 540
   Gly Arg Gly Tyr Asn Tyr Ala Thr Cys Leu Glu Gly Ala Leu Lys Ile
   545 550 555 560
   Lys Glu Ile Thr Tyr Met His Ser Glu Gly Ile Leu Ala Gly Glu Leu
   565 570 575
   Lys His Gly Pro Leu Ala Leu Ile Asp Lys Gln Met Pro Val Ile Met
   580 585 590
   Val Ile Met Lys Asp Pro Cys Phe Ala Lys Cys Gln Asn Ala Leu Gln
   595 600 605
   Gln Val Thr Ala Arg Gln Gly Arg Pro Ile Ile Leu Cys Ser Lys Asp
   610 615 620
   Asp Thr Glu Ser Ser Lys Phe Ala Tyr Lys Thr Ile Glu Leu Pro His
   625 630 635 640
   Thr Val Asp Cys Leu Gln Gly Ile Leu Ser Val Ile Pro Leu Gln Leu
   645 650 655
   Leu Ser Phe His Leu Ala Val Leu Arg Gly Tyr Asp Val Asp Phe Pro
   660 665 670
   Arg Asn Leu Ala Lys Ser Val Thr Val Glu
   675 680